(19) Europäisches Patentamt / European Patent Office / Office européen des brevets

(11) **EP 4 640 823 A1**

(12) **EUROPEAN PATENT APPLICATION**
published in accordance with Art. 153(4) EPC

(43) Date of publication:
**29.10.2025 Bulletin 2025/44**

(21) Application number: **24736942.4**

(22) Date of filing: **02.01.2024**

(51) International Patent Classification (IPC):
*C12N 1/20* (2006.01)    *A61P 35/00* (2006.01)
*A61P 3/06* (2006.01)    *C12R 1/01* (2006.01)

(52) Cooperative Patent Classification (CPC):
**A61P 3/06; A61P 35/00; C12N 1/20;** C12R 2001/01

(86) International application number:
**PCT/CN2024/070226**

(87) International publication number:
**WO 2024/141107 (04.07.2024 Gazette 2024/27)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: 30.12.2022  CN 202211737260
30.12.2022  CN 202211738395

(71) Applicant: **Moon (Guangzhou) Biotech Co., Ltd.
Guangzhou, Guangdong 510535 (CN)**

(72) Inventors:
• **XIAN, Yibo
Guangzhou, Guangdong 510535 (CN)**
• **LIU, Zhenzhen
Guangzhou, Guangdong 510535 (CN)**
• **HUANG, Baojia
Guangzhou, Guangdong 510535 (CN)**
• **KONG, Ping
Guangzhou, Guangdong 510535 (CN)**

(74) Representative: **Prüfer & Partner mbB
Patentanwälte · Rechtsanwälte
Sohnckestraße 12
81479 München (DE)**

Remarks:
The complete document including Reference
Table(s) and the Sequence Listing(s) can be
downloaded from the EPO website

(54) **NOVEL STRAIN OF COLLINSELLA, AND PHARMACEUTICAL COMPOSITION THEREOF**

(57)    Provided is a novel strain of Collinsella, and a pharmaceutical composition thereof. After screening, the obtained Gram-negative Collinsella is named Collinsella sp.MNH 18574, and after a routine species identification, it is determined that the bacterium is a new strain of Collinsella. Metabolite prediction and utilization of tumor models proves that the bacterium can improve the expression of multiple inflammatory-inducing factors, reduce the expression of anti-inflammatory factors, and can significantly improve the survival conditions of various tumor mouse models (such as lung cancer, pancreatic cancer etc.), and the bacterium can be used for developing drugs for preventing or treating various cancers. Metabolic experiments such as mouse liver and kidney function experiments and mouse obesity intervention experiments prove that the bacterium can improve and prevent metabolic diseases.

Tumor growth inhibition rate at endpoint

Fig. 14

**Description**

**TECHNICAL FIELD**

The present disclosure relates to the technical field of microorganisms, in particular to a novel species of *Collinsella* and a pharmaceutical composition thereof.

**BACKGROUND OF THE INVENTION**

The gastrointestinal (GI) tract and other organ systems are complex biological systems that comprise communities of many different organisms, including a wide variety of bacterial strains. Hundreds of different species may form symbiotic communities in the GI tract and other organs of healthy individuals. In addition, the microorganisms present in the gut not only play a crucial role for health of the digestive system, but also affect the immune system. Recent studies have shown that the gut microbiome of cancer patients has a significant influence on the efficacy of tumor immunotherapy, and that modulation and utilization of gut microbes have become key components of tumor immunotherapy. Multiple mechanisms of direct interaction between gut microbes and immune cells have been demonstrated. Dendritic cells and macrophages in intestinal lymphoid tissues are key target cells for immunomodulatory microorganisms. Particularly in the small intestine, the large intestinal surface area and the thin and diffuse mucous layer allow microorganisms to come into close contact with immune cells. Different specific strains in the microflora can inhibit or activate immune responses throughout the body. In the prior art, such as in the field of probiotics or FMT transplantation, usually a mixture of multiple microorganisms acts on the gut for metabolism and treatment of obesity. However, the mechanism of action of multiple bacteria on indications is more complex, and the influence of bacteria on each other has not been well studied. Use of multiple bacteria as a viable bacterial drug will disrupt the homeostasis of the intestinal flora. In contrast, a single bacterium has less impact on the homeostasis of the intestinal flora. In addition, the use of mixed bacteria requires additional consideration of whether the bacteria will affect each other's activities, and it is also not clear whether the synergistic effect of the mixed bacteria or a particular single bacterium plays a role in the treatment or prevention of a disease.

In recent years, more and more attention has been paid to the development of Live Biotherapeutic Products (LBPs) based on viable bacteria. Bacteria will be exposed to a rather harsh environment after they enter the digestive tract. The low pH of gastric acid in the stomach and a certain concentration of bile salts in the intestines are very harmful to many bacteria. The tolerance of a bacterium to the digestive tract environment is of great importance to its chance of being developed into a Live Biotherapeutic Product. These indicators are important in the selection of dosage forms for the development of Live Biotherapeutic Products based on this bacterial strain.

To date, 13 species and 1 subspecies have been identified under the genus *Collinsella.* However, researches mainly focus on the development and utilization of *Collinsella aerofaciens* so far. For example, vesicles from Gram-positive bacteria have peptidoglycan and lipoteichoic acid in addition to proteins and nucleic acids. The vesicles of *Collinsella aerofaciens* have been used to diagnose various cancers based on metagenomic analysis. *Collinsella aerofaciens Subsp.* (Shenzhenensis Subsp) has been found to be a butyrate-producing bacterium, which can biosynthesize butyrate and alleviate inflammatory diseases. However, the vast majority of *Collinsella* strains have not been found to be effective in the prevention or treatment of cancers. It is a great challenge for LBP research and development companies to screen out a new species of the genus *Collinsella* that can be effectively used to prevent or treat a cancer.

PD-1 inhibitors are anti-tumor inhibitors commonly used in clinical practice at present. However, it has been reported that the high expression of PD-L1 will lead to continuous activation of the PD-1/PDL1 signaling pathway, which causes tumor cells to evade the surveillance and killing of the immune system, thereby promoting tumor growth and exacerbating the drug resistance of tumors. Currently, there are rare reports on using a bacterium to inhibit tumors from acquiring drug resistance depending on the PD-1/PD-L1 signaling pathway. It is possible to investigate whether there are new bacterial strains that function to inhibit the drug resistance of tumors, thereby providing a new idea for the research and development of Live Biotherapeutic Products for tumors.

To date, the prevalence of obesity, metabolic diseases, fatty liver, or the like has been increasing. Furthermore, some studies suggest that such diseases are associated with the ecological dysregulation of microbiota. It has been reported that the abundance of *Collinsella* sp. decreases in normal-weight children, and is higher in obese children, such that *Collinsella* sp. can be used as a marker for the diagnosis of obesity.

To date, 13 species and 1 subspecies have been identified under the genus *Collinsella*. The majority of *Collinsella* species are Gram-positive and a very few of them are Gram-negative bacteria. To date, only a limited number of *Collinsella* species have been effectively developed into drugs. Therefore, it is a great challenge for LBP research and development companies to screen out a new species of the genus *Collinsella* that can be effectively used to prevent or treat fatty liver and obesity, and reduce cholesterol.

In view of this, the present invention is proposed.

SUMMARY **OF THE INVENTION**

One of the objectives of the present disclosure is to provide a new species of *Collinsella* with the function of inhibiting tumor growth, and to attempt to use the species or a secretion or metabolite obtained from its culture for the development of a corresponding anti-tumor drug, in the expectation of providing a safe and effective drug for patients with various tumors.

Another objective of the present disclosure is to provide a new species of *Collinsella* for treating metabolic diseases, and to attempt to use the species or a secretion or metabolite obtained from its culture for the development of a corresponding drug for treating metabolic diseases, in the expectation of providing a safe and effective drug for patients with metabolic diseases.

In order to solve the above technical problems and realize the above objectives, the present disclosure provides the following technical solutions:

In a first aspect, the present disclosure provides a microbial strain, wherein the microbial strain is a new species of the genus *Collinsella* (*Collinsella* sp.), and is useful for the prevention and/or treatment of a tumor; and wherein the nucleotide sequence of 16s rDNA of the microbial strain has a similarity of more than 98.65% to the nucleotide sequence as set forth in SEQ ID No: 1.

Preferably, the nucleotide sequence of 16s rDNA of the microbial strain has a similarity of greater than or equal to 98.7%, 99%, 99.3%, 99.5%, 99.8%, 99.9%, or 100% to the nucleotide sequence as set forth in SEQ ID No: 1.

In an alternative embodiment, the microbial strain comprises *Collinsella* sp. MNH 18574, which was deposited with the Guangdong Microbial Culture Collection Center with a deposit name of *Collinsella* sp. MNH 18574 and a deposit number of GDMCC No: 62667. The deposit address is Guangdong Institute of Microbiology, 5th Floor, No.59 Building, No. 100 Xianlie Zhong Road, Guangzhou. The taxonomic name is *Collinsella* sp.

Preferably, the microbial strain has an average nucleotide identity (ANI) value of at least 81.5% to the *Collinsella* strain with a deposit number of GDMCC No: 62667.

Preferably, the average nucleotide identity (ANI) value is 82%, 85%, 90%, 92%, 95%, 95.5%, 96%, 96.5%, 97%, 97.5%, 98%, 98.5%, 99%, 99.5%, 99.6%, 99.7%, 99.8%, 99.9%, or 100%.

In a second aspect, the present disclosure provides a composition, comprising at least one of the microbial strain as described in the preceding first aspect, a metabolite of the microbial strain, a fermentation culture broth of the microbial strain, a supernatant of a fermentation culture broth of the microbial strain, or any combination thereof.

Preferably, the metabolite of the microbial strain comprises at least one of (a) to (d): (a) acetic acid or acetate, (b) propionic acid or propionate, (c) butyric acid or butyrate, and (d) valeric acid or valerate.

Preferably, the microbial strain is highly productive of acetic acid. The term "highly productive" as used herein means that the yield of a short-chain fatty acid in the metabolite reaches more than 200 µg/g. It was identified that the metabolite of the *Collinsella* sp. obtained by the present disclosure contained 692.03 µg/g of acetic acid, and therefore the strain is regarded as being highly productive of acetic acid.

Optionally, the fermentation culture broth or supernatant of the fermentation culture broth is a fermentation culture broth or a supernatant of a fermentation culture broth obtained under anaerobic cultivation conditions using a liquid culture medium. Preferably, the liquid culture medium is MM01 liquid medium. Preferably, the cultivation conditions comprise: a culture temperature of 30-42 °C, e.g., 30 °C, 31 °C, 32 °C, 33 °C, 34 °C, 35 °C, 36 °C, 37 °C, 38 °C, 39 °C, 40 °C, 41 °C, 42 °C, or any value therebetween; and/or a pH of the medium of 5-10, preferably pH 6-8, e.g., 6, 6.1, 6.2, 6.3, 6.4, 6.5, 6.6, 6.7, 6.8, 6.9, 7, 7.1, 7.2, 7.3, 7.4, 7.5, 7.6, 7.7, 7.8, 7.9, 8, or any value therebetween.

Optionally, the composition can be a pharmaceutical composition, a health care product, or a food product.

The composition provided herein can be formulated into a food product. In addition to therapeutic effects, the food product can provide nutritional benefits, such as nutritional supplementation. Similarly, the composition of the present disclosure can be formulated into a food product to enhance its flavor, or it can be rendered more similar to a common food product rather than a pharmaceutical composition such that the composition is more attractive to be consumed. In certain embodiments, the composition of the present disclosure is formulated into a milk-based product. The milk-based product can be, for example, cow's milk, goat's milk, sheep's milk, skim milk, whole milk, reconstituted milk of milk powder and whey without any processing, or processed products such as yogurt, curd, full-fat yogurt, buttermilk and other yogurt products. Another important group includes milk beverage, such as whey beverage, fermented milk, condensed milk, infant or baby milk; flavored milk, ice cream; and milk-containing foods, such as confections.

The compositions of the present disclosure can be administered as a food product, such as a nutritional supplement. Generally, the composition of the present disclosure is used for the treatment of humans, although they can be used for the treatment of animals, including monogastric mammals, such as poultry, pigs, cats, dogs, horses, or rabbits. The composition of the present disclosure can be used to enhance the growth and performance of animals. If it is administered to an animal, oral administration by gavage can be used.

Optionally, the composition further comprises at least one probiotic. In some embodiments, the at least one probiotic is selected from *Bifidobacterium, Lactobacillus, Lacticaseibacillus, Limosilactobacillus, Lactiplantibacillus,*

*Ligilactobacillus, Latilactobacillus, Streptococcus, Lactococcus, Propionibacterium, Acidipropionibacterium, Leuconostoc, Pediococcus, Weizmannia, Mammaliicoccus, Staphylococcus,* and *Kluyveromyces.*

Optionally, in some embodiments, the at least one probiotic is selected from *Bifidobacterium adolescentis, Bifidobacterium animalis* subsp. *animalis, Bifidobacterium animalis* subsp. *lactis, Bifidobacterium bifidum, Bifidobacterium breve, Bifidobacterium longum* subsp. *longum, Bifidobacterium longum* subsp. *infantis, Lactobacillus acidophilus, Lactobacillus crispatus, Lactobacillus delbrueckii* subsp. *Bulgaricus, Lactobacillus delbrueckii* subsp. *Lactis, Lactobacillus gasseri, Lactobacillus helveticus, Lactobacillus johnsonii, Lactobacillus kefiranofaciens* subsp. *Kefiranofaciens, Lacticaseibacillus casei, Lacticaseibacillus paracasei, Lacticaseibacillus rhamnosus, Limosilactobacillus fermentum, Limosilactobacillus reuteri, Lactiplantibacillus plantarum, Ligilactobacillus salivarius, Latilactobacillus curvatus, Latilactobacillus sakei, Streptococcus salivarius subsp.thermophilus, Lactococcus lactis* subsp. *lactis, Lactococcus lactis* subsp. *lactis biovar diacetylactis, Lactococcus cremoris, Propionibacterium freudenreichii* subsp. *shermanii, Acidipropionibacterium acidipropionici, Leuconostoc mesenteroides* subsp. *mesenteroides, Pediococcus acidilactici, Pediococcus pentosaceus, Weizmannia coagulans, Mammaliicoccus vitulinus, Staphylococcus xylosus, Staphylococcus carnosus,* and *Kluyveromyces marxianus.*

Optionally, the composition can be used to prevent or treat a tumor or an inflammatory disease.

Optionally, the tumor is selected from a solid tumor, a soft tissue tumor, a hematopoietic tumor, an adenoma, or a metastatic tumor.

Optionally, the tumor is selected from one or more of liver cancer, colon cancer, rectal cancer, colorectal cancer, lung cancer, breast cancer, cervical cancer, ovarian cancer, pancreatic cancer, bile duct cancer, kidney cancer, glioma, liposarcoma, chondrosarcoma, fibrosarcoma, melanoma, cervical cancer, neuroblastoma, lymphoma, leukemia, gastric cancer, hematological malignancy, prostate cancer, small cell lung cancer, squamous cell carcinoma, head and neck tumor, bladder cancer, nasopharyngeal cancer, and multiple myeloma.

Optionally, the tumor is selected from a tumor caused by a virus or a tumor caused by a bacterium, such as a tumor caused by human papilloma virus (HPV) infection, hepatitis B virus (HBV) infection or hepatitis C virus (HCV) infection, human immunodeficiency virus (HIV) infection, *Helicobacter pylori* (*H. pylor*) infection, Epstein-Barr virus infection, or *Fusobacterium nucleatum* (*F. nucleatum*) infection.

Optionally, the tumor comprises a clinically refractory tumor or a drug-resistant tumor.

Optionally, the clinically refractory tumor comprises a tumor with a low response to a PD-1 drug; the drug-resistant tumor comprises a tumor resistant to a PD-1 drug and/or a tumor resistant to a chemical agent, a chemotherapeutic agent, and/or an immunotherapeutic agent; and the clinically refractory tumor or drug-resistant tumor comprises at least one of lung cancer, liver cancer, and pancreatic cancer.

Optionally, the tumor comprises a tumor resistant to an immunotherapeutic agent, such as a tumor resistant to an anti-PD-1 antibody, an anti-CTLA-4 antibody, an anti-PD-L1 antibody, and/or a PD-L1 inhibitor.

Optionally, the tumor is selected from one or more of liver cancer, colon cancer, rectal cancer, colorectal cancer, lung cancer, breast cancer, cervical cancer, ovarian cancer, pancreatic cancer, bile duct cancer, kidney cancer, glioma, liposarcoma, chondrosarcoma, fibrosarcoma, melanoma, cervical cancer, neuroblastoma, lymphoma, leukemia, gastric cancer, hematological malignancy, prostate cancer, small cell lung cancer, squamous cell carcinoma, head and neck tumor, bladder cancer, nasopharyngeal cancer, and multiple myeloma. Still preferably, the tumor is selected from one or more of melanoma, glioma, head and neck tumor, lung cancer, colorectal cancer, bladder cancer, nasopharyngeal cancer, liver cancer, kidney cancer, pancreatic cancer, lymphoma (preferably cutaneous T-cell lymphoma or peripheral T-cell lymphoma), ovarian cancer, fibrosarcoma, and multiple myeloma.

Optionally, the inflammatory disease is selected from a rheumatic disease, a connective tissue disease, systemic vasculitis, a dermatologic inflammatory disease, sepsis/systemic inflammatory response syndrome, acute respiratory distress syndrome, gastritis, pneumonia, or hepatitis. Preferably, the inflammatory disease is selected from rheumatoid arthritis, systemic sclerosis, systemic lupus erythematosus, Sjogren's syndrome, Reiter syndrome, systemic juvenile idiopathic arthritis, spondyloarthropathy, giant cell arteritis, polymyalgia rheumatica, aortitis, polyarteritis nodosa, Kawasaki disease, ANCA-associated vasculitis, immune-complex-mediated vasculitis, Behcet syndrome, relapsing polychondritis, sarcoidosis, psoriasis, psoriatic arthritis, eczema, chronic urticaria, neutrophilic dermatosis, acute or chronic gastritis, alopecia areata, asthma, bronchitis, or alcoholic or autoimmune hepatitis.

Optionally, the pharmaceutical composition further comprises at least one additional agent, which is selected from a chemotherapeutic agent, a radiotherapeutic agent, a targeted agent, a photosensitizer, a photothermal agent, an immunotherapeutic agent, or any combination thereof.

In some embodiments, the chemotherapeutic agent is selected from (a) alkylating agents, such as nitrogen mustards (e.g., nitrogen mustard, cyclophosphamide, ifosfamide, melphalan, or chlorambucil), ethyleneimines and methyl melamines (e.g., altretamine or thiotepa), alkyl sulfonates (e.g., busulfan), nitrosoureas (e.g., carmustine, lomustine, chlorozotocin, or streptozotocin), triazines (e.g., dacarbazine); (b) antimetabolites, such as folic acid analogs (e.g., methotrexate), pyrimidine analogs (e.g., gemcitabine, capecitabine, 5-fluorouracil, fluorouracil, cytarabine, or azauracil), and purine analogs and related substances (e.g., 6-mercaptopurine, 6-thioguanine, or pentostatin); (c) natural products, such as vinca alkaloids (e.g., vinblastine or vincristine), epipodophyllotoxins (e.g., etoposide or teniposide), antibiotics (e.g., actinomycin D, daunorubicin, doxorubicin, mitomycin, epirubicin, bleomycin, plicamycin, or mitoxantrone), enzymes (e.g., L-asparaginase), biological response modifiers (e.g., interferon-$\alpha$), or (d) other agents, such as platinum coordinated complexes (e.g., cisplatin or carboplatin), substituted ureas (e.g., hydroxyurea), methylhydrazine derivatives (e.g., procarbazine), and adrenocortical inhibitors (e.g., paclitaxel or mitotane). In some embodiments, the chemotherapeutic agent comprises at least one of gemcitabine, oxaliplatin, paclitaxel, camptothecin, 5-fluorouracil, cisplatin, doxorubicin, mitomycin, or epirubicin.

In some embodiments, the radiotherapeutic agent comprises an agent used in external radiotherapy, internal radiotherapy, radioimmunotherapy, or intraoperative radiotherapy (IORT).

In some embodiments, the photosensitizer comprises at least one of boron dipyrrole, dihydroporphin, or Rose-Bengal.

In some embodiments, the photothermal agent comprises at least one of indocyanine green, new indocyanine green, or gold nanoparticle rods.

In some embodiments, the immunotherapeutic agent comprises an immune checkpoint inhibitor, a co-stimulatory molecule inhibitor, dendritic cells, CAR-T cells, a cytokine, or adoptive T cells. In some embodiments, the immune checkpoint inhibitor is selected from an inhibitor capable of binding, blocking, and/or inhibiting the activity of one or more of CTLA-4, PDL1, PDL2, PD1, BTLA, HVEM, TIM3, GAL9, LAG3, VISTA, KIR, 2B4, CD160, and CGEN-15049. In some embodiments, the immune checkpoint inhibitor comprises at least one of an anti-PD-1 antibody, an anti-CTLA-4 antibody, an anti-PD-L1 antibody, or a PD-L1 inhibitor. In some embodiments, the immune checkpoint inhibitor is selected from durvalumab, atezolizumab, avelumab, pembrolizumab, nivolumab, ibritumomab, or any combination thereof. In some embodiments, the dose of the anti-PD-1 antibody is 5 to 15 mg/kg, such as 5 mg/kg, 6 mg/kg, 7 mg/kg, 8 mg/kg, 9 mg/kg, 10 mg/kg, 11 mg/kg, 12 mg/kg, 13 mg/kg, 14 mg/kg, 15 mg/kg, or any value therebetween.

Optionally, the composition is used for the prevention or treatment of metabolic diseases.

Optionally, the metabolic disease comprises at least one of obesity, an obesity-related disease, diabetes, a diabetes-related disease, a liver function disorder, a liver and kidney function disorder, inflammation, a metabolic disorder caused by obesity, a cardiovascular or cerebrovascular disease, dyslipidemia, abnormal lipid metabolism, hypercholesterolemia, or atherosclerosis.

Optionally, the composition further comprises at least one additional agent, which is selected from antidiabetic drugs.

Optionally, the antidiabetic drug comprises at least one of an insulin secretion promoter, an insulin sensitizer, a glucose source reducing agent, insulin and an insulin analog, or a novel glucose-lowering drug. The insulin secretion promoter comprises at least one of sulfonylureas (e.g., gliquidone, glipizide, glibenclamide, gliclazide, or glimepiride) or meglitinides (e.g., repaglinide, nateglinide, or mitiglinide). The insulin sensitizer comprises thiazolidinediones (e.g., rosiglitazone or pioglitazone). The glucose source reducing agent comprises at least one of biguanides (e.g., metformin) and $\alpha$-glucosidase inhibitors (e.g., acarbose, voglibose, or miglitol). The insulin and insulin analog comprises at least one of ultra-short-acting insulin analogs, mainly including aspart (insulin aspart, marketed as NovoRapid), lispro (insulin lispro, marketed as Humalog), or glulisine (Insulin glulisine, marketed as Apidra); short-acting insulin, including: regular insulin, biosynthesized human insulin (Novolin®, Humulin®, or Gansulin®); intermediate-acting insulin (NPH): isophane insulin, including Novolin N, Humulin N, or Gansulin N; long-acting insulin, including protamine zinc insulin (PZI), the long-acting insulin analog detemir (insulin detemir, marketed as Levemir), the long-acting insulin analog glargine (insulin glargine, marketed as Lantus or Basalin); the ultra-long-acting insulin analog Tresiba (insulin degludec); or premixed insulin, including premixed 30R, premixed 50R, premixed insulin analogs insulin aspart 30 (NovoRapid 30), premixed insulin aspart 50 (NovoRapid 50), premixed insulin lispro 25 (Humalog 25), or premixed insulin lispro 50 (Humalog 50). The novel glucose-lowering drug comprises at least one of a human glucagon-like peptide-1 (GLP-1) receptor agonist (e.g., exenatide (marketed as Byetta), Liraglutide (marketed as Victoza), or Semaglutide (marketed as Ozempic)), a dipeptidyl peptidase-4 (DPP-4) inhibitor (e.g., sitagliptin, saxagliptin, vildagliptin, alogliptin, or linagliptin), a sodium-glucose cotransporter 2 (SGLT2) inhibitor (e.g., dapagliflozin, empagliflozin, or canagliflozin), or a pancreatic amyloid polypeptide analog (e.g., pramlintide).

Optionally, the composition comprises a pharmaceutically or food-acceptable excipient.

The acceptable excipient is selected from pharmaceutically or food-acceptable excipients, diluents, or carriers. The pharmaceutically or food-acceptable excipients, diluents, or carriers are well known in the pharmaceutical field or in the food field. Examples of suitable carriers include lactose, starch, dextrose, methylcellulose, magnesium stearate, mannitol, sorbitol, etc. Examples of suitable diluents include ethanol, glycerol, and water. The pharmaceutical carrier, excipient, or diluent can be selected depending on the intended route of administration and standard pharmaceutical practice. The pharmaceutical composition can comprise any suitable binder, lubricant, suspending agent, coating agent, solubilizer, or the like as or in addition to the carrier, excipient or diluent. Examples of a suitable binder include starch, gelatin, a natural sugar, and a natural or synthetic gum. The natural sugar is, for example, glucose, anhydrous lactose, free-flowing lactose, $\beta$-lactose, or corn sweetener. The natural or synthetic gum is, for example, gum arabic, tragacanth, sodium alginate, carboxymethyl cellulose, or polyethylene glycol. Examples of a suitable lubricant include sodium oleate, sodium stearate, magnesium stearate, sodium benzoate, sodium acetate, sodium chloride, or the like. A preservative, a stabilizer, a dye, or even a flavoring agent can be provided in the pharmaceutical composition. Examples of the preservative include sodium benzoate, sorbic acid, or parabens. An antioxidant or a suspending agent can also be used. An antioxidant or a suspending agent can also be used.

Optionally, the composition has a characteristic selected from any one or more of (1) to (7) below:

(1) the composition is in a dosage form suitable for infants, children, or adults;

(2) the composition is in a dosage form for gastrointestinal administration or parenteral administration;

(3) the composition is an oral preparation or an injection;

(4) the microbial strain can at least partially proliferate in the intestinal tract of a subject;

(5) the microbial strain, or a metabolite, culture or culture supernatant thereof, is present in the pharmaceutical composition in a mass percent content of 1-80%; e.g., 2-70%, 5-60%, 10-50%, or 20-40%, specifically, e.g., 5%, 10%, 15%, 20%, 25%, 30%, 35%, 40%, 45%, 50%, 55%, 60%, 65%, 70%, 75%, 80%, or any value therebetween, preferably 45%, 50%, 55%, or 60%;

(6) the form of the microbial strain is selected from viable bacteria, attenuated bacteria, killed bacteria, lyophilized bacteria, or irradiated bacteria; and

(7) the composition comprises $1 \times 10^3$ to $1 \times 10^{13}$ colony forming units (CFUs) of the microbial strain per g of the composition; e.g., $1 \times 10^4$ to $1 \times 10^{12}$, $1 \times 10^5$ to $1 \times 10^{11}$, or $1 \times 10^6$ to $1 \times 10^{10}$ colony forming units (CFUs) of the microbial strain per g of the composition, specifically, e.g., $1 \times 10^3$, $5 \times 10^3$, $1 \times 10^4$, $5 \times 10^4$, $1 \times 10^5$, $5 \times 10^5$, $1 \times 10^6$, $5 \times 10^6$, $1 \times 10^7$, $5 \times 10^7$, $1 \times 10^8$, $5 \times 10^8$, $1 \times 10^9$, $5 \times 10^9$, $1 \times 10^{10}$, $5 \times 10^{10}$, $1 \times 10^{11}$, $5 \times 10^{11}$, $1 \times 10^{12}$, $5 \times 10^{12}$, $1 \times 10^{13}$, $5 \times 10^{13}$, or any value therebetween, colony forming units (CFUs) of the microbial strain per g of the composition.

Optionally, the pharmaceutical composition is in one of the following forms: a powder, a microencapsulated powder, a capsule, a tablet, a lozenge, a granule, an oral liquid, a suspension, an emulsion, a liquid formulation, a sustained-release formulation, a nano-formulation, and a microencapsulated capsule.

Optionally, the pharmaceutical composition provided herein is administered orally. Oral administration may involve swallowing, whereby the compound enters the gastrointestinal tract, and/or buccal, lingual or sublingual administration, whereby the compound directly enters the bloodstream from the mouth. Pharmaceutical dosage forms suitable for oral administration include solid suppositories, solid particles, semi-solids, and liquids (including multiphase or dispersible systems) such as tablets; soft or hard capsules containing multiple particles or nanoparticles, liquids (e.g., aqueous solutions), emulsions, or powders; lozenges (including a liquid filler); chewables; gels; rapidly dispersible dosage forms; beads; sprays; and buccal/mucosal adhesion patches.

Optionally, the composition is an enteric-coated formulation, i.e., a gastric fluid-tolerant (e.g., gastric pH-tolerant) formulation suitable for delivery of the composition of the present disclosure to the gut by oral administration. An enteric-coated formulation can be particularly useful when the bacterium or another component of the composition is acid-sensitive, e.g., readily degradable under gastric conditions. In some embodiments, the enteric-coated formulation comprises an enteric coating. In some embodiments, the composition is in an enteric-coated dosage form. For example, the enteric-coated formulation can be an enteric-coated granule, an enteric-coated tablet, an enteric-coated capsule, or the like. In some embodiments, the composition is a soft capsule. In some embodiments, the composition is a capsule. In some embodiments, the capsule can be a hard capsule or a soft capsule, or the capsule can be a sustained-release capsule, a controlled-release capsule, or an enteric-coated capsule, or the capsule can be a microencapsulated capsule, a microcapsule, or the like.

Optionally, the composition is a soft capsule. The soft capsule is a capsule which has a certain elasticity and softness due to the presence of a softener such as glycerol, sorbitol, maltitol, or polyethylene glycol in the capsule shell. The soft capsule can be manufactured on the basis of, for example, gelatin or starch. Gelatin-based soft capsules are commercially available from various suppliers. Depending on the mode of administration, e.g., oral or rectal administration, the soft capsules can have various shapes, which may be, for example, round, oval, elliptical, or torpedo-shaped. The soft capsule can be manufactured by a conventional process, such as the Scherer process, the Accogel process, or the dropping or blow molding process.

Optionally, the composition can be administered to the gastrointestinal tract via a tube, such as a nasal feeding tube, an orogastric tube, a gastric tube, a jejunostomy tube (J-tube), percutaneous endoscopic gastrostomy (PEG), or a port, such as a port leading to the stomach or the jejunum, or other chest wall ports suitable for access ports.

Optionally, the microbial strain in the composition is freeze-dried or spray-dried. In some embodiments, the microbial strain in the composition is spray-dried. In some embodiments, the microbial strain in the composition is electrostatically spray-dried. In some embodiments, the microbial strain in the composition is lyophilized or spray-dried, and it is alive. In some embodiments, the microbial strain in the composition is lyophilized or spray-dried, and it can partially or completely colonize the intestine. In some embodiments, the lyophilized microbial strain is reconstituted prior to administration. In some embodiments, the reconstitution is carried out by using a diluent as described herein.

Optionally, the administration of the composition is accompanied by an assessment of the patient's intestinal microbiota. If the delivery and/or partial or complete colonization of the strain of the present disclosure are not achieved and thus no efficacy is observed, then the treatment can be repeated. If the delivery and/or partial or complete colonization are successful and an efficacy is observed, then the treatment can be discontinued.

In a third aspect, the present disclosure provides use of the microbial strain as described in the first aspect and/or the composition as described in the second aspect in the manufacture of a medicament for preventing or treating a tumor.

Optionally, the tumor is selected from a solid tumor, a soft tissue tumor, a hematopoietic tumor, an adenoma, or a metastatic tumor.

Optionally, the tumor is selected from one or more of liver cancer, colon cancer, rectal cancer, colorectal cancer, lung cancer, breast cancer, cervical cancer, ovarian cancer, pancreatic cancer, bile duct cancer, kidney cancer, glioma, liposarcoma, chondrosarcoma, fibrosarcoma, melanoma, cervical cancer, neuroblastoma, lymphoma, leukemia, gastric cancer, hematological malignancy, prostate cancer, small cell lung cancer, squamous cell carcinoma, head and neck tumor, bladder cancer, nasopharyngeal cancer, and multiple myeloma.

Optionally, the tumor is selected from a tumor caused by a virus or a tumor caused by a bacterium, such as a tumor caused by human papilloma virus (HPV) infection, hepatitis B virus (HBV) infection or hepatitis C virus (HCV) infection, human immunodeficiency virus (HIV) infection, *Helicobacter pylori* (*H. pylor*) infection, Epstein-Barr virus infection, or *Fusobacterium nucleatum* (*F. nucleatum*) infection.

Optionally, the tumor comprises a clinically refractory tumor or a drug-resistant tumor.

Optionally, the clinically refractory tumor comprises a tumor with a low response to a PD-1 drug; the drug-resistant tumor comprises a tumor resistant to a PD-1 drug and/or a tumor resistant to a chemical agent, a chemotherapeutic agent, and/or an immunotherapeutic agent; and the clinically refractory tumor or drug-resistant tumor comprises at least one of lung cancer, liver cancer, and pancreatic cancer.

Optionally, the tumor comprises a tumor resistant to an immunotherapeutic agent, such as a tumor resistant to an anti-PD-1 antibody, an anti-CTLA-4 antibody, an anti-PD-L1 antibody, and/or a PD-L1 inhibitor.

Optionally, the tumor is selected from one or more of liver cancer, colon cancer, rectal cancer, colorectal cancer, lung cancer, breast cancer, cervical cancer, ovarian cancer, pancreatic cancer, bile duct cancer, kidney cancer, glioma, liposarcoma, chondrosarcoma, fibrosarcoma, melanoma, cervical cancer, neuroblastoma, lymphoma, leukemia, gastric cancer, hematological malignancy, prostate cancer, small cell lung cancer, squamous cell carcinoma, head and neck tumor, bladder cancer, nasopharyngeal cancer, and multiple myeloma. Still preferably, the tumor is selected from one or more of melanoma, glioma, head and neck tumor, lung cancer, colorectal cancer, bladder cancer, nasopharyngeal cancer, liver cancer, kidney cancer, pancreatic cancer, lymphoma (preferably cutaneous T-cell lymphoma or peripheral T-cell lymphoma), ovarian cancer, fibrosarcoma, and multiple myeloma.

Optionally, the medicament is used to enhance the efficacy of one or more anti-tumor therapies.

Optionally, the anti-tumor therapy comprises use of one or more chemotherapeutic agents, radiotherapeutic agents, targeted agents, chemotherapeutic photosensitizers, photothermal agents, and/or immunotherapies.

Optionally, the medicament can prevent or treat a tumor by at least one of the following means:
(a) inhibiting tumor volume growth; (b) inhibiting tumor weight increase; (c) inhibiting tumor cell growth; (d) improving the tumor response rate to a therapy; (e) improving the therapeutic efficacy of an immunosuppressive agent, e.g., improving the therapeutic efficacy of a PD-1 drug; (f) inhibiting tumor cell metastasis; (g) reducing the resistance to a PD-1 drug; (h) inhibiting the tumor by at least one short-chain fatty acid or short-chain fatty acid salt of acetic acid or acetate, propionic acid or propionate, butyric acid or butyrate, valeric acid or valerate in SCFA; (i) inhibiting the tumor by regulating the subject's immune system to exert an immunomodulatory effect; or (j) promoting or activating the subject's immune system (e.g., increasing $CD4^+$ or $CD8^+$ T cell content) to kill the tumor or inhibit tumor growth.

In some embodiments, the medicament inhibits the tumor growth by immunomodulating macrophages, primary PBMCs, and/or monocyte-derived dendritic cells. In some embodiments, the *Collinsella* sp. can increase the production of one or more pro-inflammatory cytokines and/or chemokines by human macrophages, monocytes, peripheral blood mononuclear cells, and/or monocyte-derived dendritic cells, and/or can increase the infiltration of T-cells in the tumor. In some embodiments, the pro-inflammatory cytokine is selected from one or more of interferon $\gamma$, interleukin IL-1$\beta$, IL-6, IL-12, IL-23P40, or tumor necrosis factor TNF$\alpha$; and/or the chemokine is selected from one or more of MCP-1 (CCL2), MIG (CXCL9), RANTES (CCL5), IP-10 (CXCL-10), or IL-8. In some embodiments, the immunomodulation of macrophages by the medicament is induction of differentiation of M0-type macrophages into M1-type macrophages by the *Collinsella* sp.; wherein the levels of the pro-inflammatory factors IL-1β, IL-6, and TNFα are significantly upregulated in the macrophages, and/or the levels of the chemokines MIG and MCP-1 are significantly upregulated in the macrophages. In some embodiments, the immunomodulation of primary PBMCs by the medicament is induction of an increase in the expression of the chemokines IP-10, MCP-1, MIG, and RANTES in the primary PBMCs by the *Collinsella* sp.; and/or induction of an increase in the expression of the inflammatory factors IL-1β, TNFα, IL-6, and IFNγ in the primary PBMCs by the *Collinsella* sp.

In a fourth aspect, the present disclosure provides use of the microbial strain as described in the first aspect and/or the composition as described in the second aspect in the manufacture of a medicament for preventing or treating an inflammatory disease.

Optionally, the inflammatory disease is selected from a rheumatic disease, a connective tissue disease, systemic vasculitis, a dermatologic inflammatory disease, sepsis/systemic inflammatory response syndrome, acute respiratory distress syndrome, gastritis, pneumonia, or hepatitis.

Preferably, the inflammatory disease is selected from rheumatoid arthritis, systemic sclerosis, systemic lupus erythematosus, Sjogren's syndrome, Reiter syndrome, systemic juvenile idiopathic arthritis, spondyloarthropathy, giant cell arteritis, polymyalgia rheumatica, aortitis, polyarteritis nodosa, Kawasaki disease, ANCA-associated vasculitis, immune-complex-mediated vasculitis, Behcet syndrome, relapsing polychondritis, sarcoidosis, psoriasis, psoriatic arthritis, eczema, chronic urticaria, neutrophilic dermatosis, acute or chronic gastritis, alopecia areata, asthma, bronchitis, or alcoholic or autoimmune hepatitis.

Optionally, the medicament prevents or treats the inflammatory disease by modulating the immune system.

In a fifth aspect, the present disclosure provides use of the microbial strain as described in the first aspect and/or the composition as described in the second aspect in the manufacture of a medicament for preventing or treating a metabolic disease.

Optionally, the metabolic disease comprises at least one of obesity, an obesity-related disease, diabetes, a diabetes-related disease, a liver function disorder, a liver and kidney function disorder, inflammation, a metabolic disorder caused by obesity, a cardiovascular or cerebrovascular disease, dyslipidemia, abnormal lipid metabolism, hypercholesterolemia, or atherosclerosis.

Optionally, the obesity-related disease comprises at least one of obesity, overeating, binge eating, bulimia, hypertension, diabetes mellitus, elevated plasma insulin concentration, insulin resistance, hyperlipidemia, metabolic syndrome, insulin resistance syndrome, obesity-related gastroesophageal reflux disease, atherosclerosis, hypercholesterolemia, hyperuricemia, lower back pain, cardiomegaly and left ventricular hypertrophy, lipodystrophia, non-alcoholic steatohepatitis, a cardiovascular disease, and polycystic ovary syndrome.

Optionally, the diabetes-related disease comprises at least one of type I diabetes (T1D), type II diabetes (T2D), gestational diabetes mellitus (GDM), insulin resistance syndrome, glucose intolerance, a hyperlipidemic disorder, a complication of diabetic nephropathy, diabetic neuropathy, diabetic ophthalmopathy, a cardiovascular disease, and diabetic foot.

In a sixth aspect, the present disclosure provides use of the microbial strain as described in the first aspect and/or the composition as described in the second aspect in the manufacture of a weight management composition.

Optionally, the weight management composition is used to achieve at least one of reducing body weight, maintaining body weight, controlling body weight gain, reducing body mass index (BMI), maintaining BMI, and controlling BMI increase.

In a seventh aspect, the present disclosure provides a method of preventing or treating a tumor, comprising administering to a subject in need thereof an effective amount of the microbial strain as described in the first aspect and/or the composition as described in the second aspect of the present disclosure.

In an eighth aspect, the present disclosure provides a method of preventing or treating an inflammatory disease, comprising administering to a subject in need thereof an effective amount of the microbial strain as described in the first aspect and/or the composition as described in the second aspect of the present disclosure.

In a ninth aspect, the present disclosure provides a method of preventing or treating a metabolic disease, comprising administering to a subject in need thereof an effective amount of the microbial strain as described in the first aspect and/or the composition as described in the second aspect of the present disclosure.

In a tenth aspect, the present disclosure provides a method of weight management, comprising administering to a subject in need thereof an effective amount of the microbial strain as described in the first aspect and/or the composition as described in the second aspect of the present disclosure.

A Gram-negative *Collinsella* sp. was obtained through screening in the present disclosure, and was named *Collinsella* sp. MNH 18574. This bacterium was identified as a new species of the genus *Collinsella* by conventional species identification, and by comparing the resistance genes, virulence genes, and metabolite genes of this bacterium with those of other species of the same genus *Collinsella.* The inventors predicted that the bacterium had a potential for cancer prevention or treatment based on its metabolites, and then constructed a variety of tumor models to carry out anti-cancer experiments and experiments on co-administration with other antitumor drugs. It has been demonstrated that the bacterium can increase the expression of a variety of induced inflammatory factors, reduce the expression of anti-inflammatory factors, and can significantly improve the survival of a variety of tumor model (e.g., lung tumors, liver tumors, and pancreatic tumors) mice. Furthermore, it can also be used in co-administration, proving that the new species provided herein can be used in the development of drugs for preventing or treating a variety of cancers.

Through metabolic experiments such as liver and kidney function experiments in mice and obesity intervention experiments in mice, it has been proved that the bacterium can improve and prevent metabolic diseases, and is expected to be used in the development of Live Biotherapeutic Products.

In addition, the following was validated in the present disclosure:

(1) After inoculating the lung cancer cell line LLC1 into mice subcutaneously to grow into tumors, it was demonstrated through MNH18574 intervention that MNH18574 had an obvious effect of inhibiting the growth of lung tumors.

(2) By using MNH18574 in combination with an anti-PD-1 antibody in an ectopic syngeneic tumor model created by subcutaneously inoculating mice with the lung cancer cell line LLC1, it was demonstrated that the combination of MNH18574 with the anti-PD-1 antibody had the effects of significantly reducing tumor volume and weight, and significantly improving the response rate of the tumor to the treatment.

(3) By using MNH18574 in combination with an anti-PD-1 antibody in an ectopic syngeneic tumor model created by subcutaneously inoculating mice with the pancreatic cancer cell line KPC, it was demonstrated that the combination of MNH18574 with the anti-PD-1 antibody had the effects of significantly reducing tumor volume and weight, and significantly improving the response rate of the tumor to the treatment.

(4) An *In vivo* experiment in a high-fat diet-induced obese mice model proved that MNH18574 significantly reduced the liver weight, indicating that MNH18574 can significantly improve the fatty liver induced by high-fat diet; proved that MNH18574 significantly inhibited the body weight gain of high-fat diet-induced obese mice, suggesting an effect of preventing and treating obesity; proved that MNH18574 reduced total cholesterol and low density lipoprotein cholesterol levels in high-fat diet-induced obese mice, suggesting an effect of preventing and treating hyperlipidemia; and proved that MNH18574 significantly reduced the area under the curve of oral glucose tolerance test (OGTT) (AUC of OGTT) in high-fat diet-induced obese mice, suggesting an effect of preventing and treating diabetes mellitus, and improving the blood glucose control function.

## BRIEF DESCRIPTION OF THE DRAWINGS

In order to more clearly illustrate the technical solutions in the specific embodiments of the present disclosure or the prior art, the accompanying drawings to be used in the description of the specific embodiments or the prior art will be briefly introduced below. Obviously, the accompanying drawings in the following description relate to some embodiments of the present disclosure. For those of ordinary skill in the art, other drawings can be obtained based on these accompanying drawings without exerting any creative effort.

Fig. 1 shows the colonial morphology of the strain MNH 18574 in Example 1 of the present disclosure;

Fig. 2 shows the Gram staining results of the strain MNH 18574 in Example 1 of the present disclosure;

Fig. 3 is a micrograph showing the morphology of the strain MNH 18574 in Example 1 of the present disclosure;

Fig. 4 shows the results from the pH tolerance test of the strain MNH 18574 in Example 1 of the present disclosure;

Fig. 5 shows the results from the NaCl tolerance test of the strain MNH 18574 in Example 1 of the present disclosure;

Fig. 6 shows the results from the bile salt tolerance test of the strain MNH 18574 in Example 1 of the present disclosure;

Fig. 7 shows the results from the API 20A test of the strain MNH 18574 in Example 1 of the present disclosure;

Fig. 8 shows the phylogenetic tree of the strain MNH 18574 in Example 1 of the present disclosure;

Fig. 9 shows the results from the macrophage immunoreactivity regulation experiment of the strain MNH 18574 in Example 2 of the present disclosure;

Fig. 10 shows the results from the Primary PBMC immunoreactivity regulation experiment of the strain MNH 18574 in Example 2 of the present disclosure;

Fig. 11 shows the tumor volume curves of mice in the experiment for verifying the therapeutic effect of the strain MNH 18574 in combination with an anti-PD-1 antibody on lung cancer;

Fig. 12 shows a comparison of tumor volumes of mice at the experimental endpoint in different experimental groups in the experiment for verifying the therapeutic effect of MNH18574 in combination with an anti-PD-1 antibody on lung cancer;

Fig. 13 shows a comparison of tumor weights of mice at the experimental endpoint in different experimental groups in the experiment for verifying the therapeutic effect of MNH18574 in combination with an anti-PD-1 antibody on lung cancer;

Fig. 14 shows a comparison of tumor growth inhibition rates of mice at the experimental endpoint in different experimental groups in the experiment for verifying the therapeutic effect of MNH18574 in combination with an anti-PD-1 antibody on lung cancer;

Fig. 15 shows a comparison of response rates of mice at the experimental endpoint in different experimental groups in the experiment for verifying the therapeutic effect of MNH18574 in combination with an anti-PD-1 antibody on lung cancer;

Fig. 16 shows the tumor volume curves of mice in the experiment for verifying the therapeutic effect of the stain MNH18574 in combination with an anti-PD-1 antibody on pancreatic cancer;

Fig. 17 shows a comparison of tumor volumes of mice at the experimental endpoint in different experimental groups in the experiment for verifying the therapeutic effect of MNH18574 in combination with an anti-PD-1 antibody on pancreatic cancer;

Fig. 18 shows a comparison of tumor weights of mice at the experimental endpoint in different experimental groups in the experiment for verifying the therapeutic effect of MNH18574 in combination with an anti-PD-1 antibody on pancreatic cancer;

Fig. 19 shows a comparison of tumor growth inhibition rates of mice at the experimental endpoint in different experimental groups in the experiment for verifying the therapeutic effect of MNH18574 in combination with an anti-PD-1 antibody on pancreatic cancer;

Fig. 20 shows a comparison of response rates of mice at the experimental endpoint in different experimental groups in the experiment for verifying the therapeutic effect of MNH18574 in combination with an anti-PD-1 antibody on pancreatic cancer;

Fig. 23 shows the experimental results of the strain MNH 18574 in reducing liver weight;

Fig. 24 shows the effect of the strain MNH 18574 on the body weight of mice in the obesity model;

Fig. 25 shows the effect of the strain MNH 18574 on the serum low density lipoprotein cholesterol level in high-fat diet-induced obesity model mice; and

Fig. 26 shows the effect of the strain MNH 18574 on the area under the curve of oral glucose tolerance test (OGTT) (AUC of OGTT) in high-fat diet-induced obese mice.

## DETAILED DESCRIPTION OF THE INVENTION

The technical solutions of the present disclosure will be clearly and completely described below with reference to the Examples. Obviously, the described embodiments are part of the embodiments of the present disclosure rather than all the embodiments. All other embodiments obtained by those of ordinary skill in the art based on the embodiments of the present disclosure without exerting any creative effort fall within the scope of protection of the present disclosure.

Unless otherwise defined, all technical and scientific terms used in the present disclosure have the same meaning as commonly used in the field to which the present disclosure belongs. For the purpose of interpreting this specification, the following definitions will be applied and, where appropriate, the terms presented in singular form will also include the plural situation and vice versa.

As used herein, the expression "a", "an", or a specific reference without a number includes plural references, unless the context clearly indicates otherwise.

The *Collinsella* sp. MNH 18574 used in the present disclosure was isolated from a stool sample from a healthy volunteer in Tibet, and was deposited on August 4, 2020 with the Guangdong Microbial Culture Collection Center with a deposit name of *Collinsella* sp. MNH 18574 and a deposit number of GDMCC 62667. The deposit address is Guangdong Institute of Microbiology, 5th Floor, No.59 Building, No. 100 Xianlie Zhong Road, Guangzhou. The test result showed that it was viable. The taxonomic name is *Collinsella* sp.

The strain has the following exemplary characteristics:

(1) The *Collinsella* sp. MNH 18574 has a 16s rRNA sequence as set forth in SEQ ID NO: 1.

(2) The *Collinsella* sp. MNH 18574 has the following physiological and biochemical characteristics: the *Collinsella* sp. MNH 18574 can grow at a temperature ranging from 30 to 42 °C, with an optimal growth temperature of 37 °C. It can grow at pH 5.0-10.0, with an optimal growth pH of 6.0-8.0. It can tolerate up to 1% NaCl. The strain MNH 18574 can survive and grow at a bile salt concentration in the range of 0-0.30%, and cannot grow at a bile salt concentration greater than or equal to 0.40%. The strain MNH 18574 cannot grow under aerobic conditions, but grows well under anaerobic conditions, and thus is an obligate anaerobic bacterium.

(3) The *Collinsella* sp. MNH 18574 is Gram-negative.

(4) The *Collinsella* sp. MNH 18574 has an ANI value ≤ 85.41% to other strains of the genus *Collinsella*.

As used herein, the term "metabolite" refers to a compound, composition, or molecule that is used as a substrate or product in any cellular or microbial metabolic reaction, or an ion, cofactor, catalyst or nutrient from any cellular or microbial metabolic reaction.

The term "strain" refers to a member of a bacterial species that has a genetic characteristic such that it can be distinguished from closely related members of the same bacterial species. The genetic characteristic can be the absence of all or part of at least one gene, the absence of all or part of at least one regulatory region (e.g., promoter, terminator, riboswitch, or ribosome binding site), the absence ("curing" of at least one natural plasmid), the presence of at least one recombinant gene, the presence of at least one mutated gene, the presence of at least one exogenous gene (a gene from another species), at least one mutated regulatory region (e.g., promoter, terminator, riboswitch, or ribosome binding site), the presence of at least one non-natural plasmid, the presence of at least one antibiotic resistance cassette, or a combination thereof. The genetic characteristic between different strains can be identified by PCR amplification, optionally followed by DNA sequencing of the genomic region of interest or the entire genome. In cases where one strain (as compared to another strain of the same species) acquires or loses antibiotic resistance or acquires or loses biosynthesis ability (e.g., an auxotrophic strain), the strain or nutrient/metabolite can be identified by selection or counter-selection using an antibiotic.

The composition of the present disclosure comprising the *Collinsella* sp. MNH 18574, a metabolite, a fermentation culture broth, a supernatant of a fermentation culture broth thereof, or any combination thereof, can be formulated in any form suitable for enhancing the abundance of the *Collinsella* sp. in a subject.

The term "metabolite" refers to a compound, composition, or molecule that is used as a substrate or product in any cellular or microbial metabolic reaction, or an ion, cofactor, catalyst or nutrient from any cellular or microbial metabolic reaction.

The term "supernatant" refers to a culture supernatant of the bacterial strain according to the present disclosure, optionally comprising a compound and/or cellular debris of the strain, and/or a metabolite and/or molecule secreted by the strain.

In some embodiments, the medicament is administered orally (e.g., by oral gavage). In some embodiments, the medicament can be administered by a route including intramuscular, inhalation, intracranial, intralymphatic, intraocular, intraperitoneal, intrapleural, intrathecal, intratracheal, intrauterine, intravascular, intravenous, intravesical, intranasal, gastrointestinal, biliary perfusion, cardiac perfusion, pre-anal, rectal, spinal, subcutaneous, sublingual, topical, intravaginal, percutaneous, ureteral, or urethral administration. Examples of suitable dosage forms include, but are not limited to, tablets, aerosols, chewable sticks, capsules, capsules containing coated particles, capsules containing sustained-release particles, capsules containing controlled-release particles, concentrates, creams, enhanced creams, suppositories, disks, dressings, elixirs, emulsions, enemas, sustained-release fibers, sustained-release films, gases, gels, metered-dose gels, granules, sustained-release granules, powders, sustained-release powders, metered-dose powders, rings, shampoo, soap solutions, mud solutions, solutions/drops, concentrated solutions, gel-forming solutions/drops, effervescent tablets, chewing gums, implants, inhalants, injections, lipid complexes for injection, liposomes for injection, inserts, sustained-release inserts, intra-uterine devices, jellies, liquids, sustained-release liquids, lotions, enhanced lotions, oils, ointments, enhanced ointments, pastes, sponges, sprays, metered-dose sprays, suppositories, suspensions, suspensions/drops, sustained-release suspensions, swabs, syrups, tablets, chewable tablets, tablets containing coated particles, dispersible tablets, effervescent tablets, sustained-release tablets, orally disintegrating tablets, tampons, tapes, or cannulas/lozenges. In some embodiments, the medicament is a sugar-coated tablet, gel capsule, gel, emulsion, tablet, flake-like capsule, hydrogel, nanofiber gel, electrospun fiber, food stick, candy, fermented milk, fermented cheese, chewing gum, powder, toothpaste, or the like. In some embodiments, the administration can also be achieved by inclusion in the subject's diet, e.g., in a functional food for use in humans or companion animals. As used herein, the subject can be human or an animal, including, but not limited to, cow, sheep, goat, cat, dog, horse, rabbit, monkey, mouse, rat, alpaca, camel, or the like.

In some embodiments, the composition provided herein is administered orally. Oral administration may involve swallowing, whereby the compound enters the gastrointestinal tract, and/or buccal, lingual or sublingual administration, whereby the compound directly enters the bloodstream from the mouth. Pharmaceutical dosage forms suitable for oral administration include solid suppositories, solid particles, semi-solids, and liquids (including multiphase or dispersible systems) such as tablets; soft or hard capsules containing multiple particles or nanoparticles, liquids (e.g., aqueous solutions), emulsions, or powders; lozenges (including a liquid filler); chewables; gels; rapidly dispersible dosage forms; beads; sprays; and buccal/mucosal adhesion patches.

In some embodiments, the composition is an enteric-coated formulation, i.e., a gastric fluid-tolerant (e.g., gastric pH-tolerant) formulation suitable for delivery of the composition of the present disclosure to the gut by oral administration. An enteric-coated formulation can be particularly useful when the bacterium or another component of the composition is acid-sensitive, e.g., readily degradable under gastric conditions.

In some embodiments, the enteric-coated formulation comprises an enteric coating. In some embodiments, the composition is in an enteric-coated dosage form. For example, the enteric-coated formulation can be an enteric-coated granule, an enteric-coated tablet, an enteric-coated capsule, or the like. The enteric-coated granule refers to a granule preparation made by coating particles with an enteric-coating material or by other suitable methods. The enteric-coated granule is resistant to gastric acid while releasing the active ingredient in intestinal fluid or controlling the site-specific release of the drug in the intestinal tract, which can prevent decomposition and failure of the drug in the stomach and avoid irritation to the stomach. The enteric-coated tablet refers to a tablet coated with an enteric-coating material. The tablet can be coated with an enteric coating in order to prevent decomposition and failure of the drug substance in the stomach, avoid irritation to the stomach, or control the site-specific release of the drug substance in the intestinal tract. Also, the tablet can be coated with a colon-sepcific enteric coating in order to treat a disease in the colon. The enteric-coated capsule refers to a hard capsule made by filling a capsule with enteric-coated granules or pellets, or a hard or soft capsule prepared with an appropriate enteric coating material. The enteric-coated capsule is not soluble in gastric fluid but can disintegrate in intestinal fluid to release the active ingredient.

In some embodiments, the composition is a capsule. The capsule refers to a solid preparation made by filling a hollow capsule or sealing in a soft capsule material with a drug substance and optionally a suitable excipient. Capsules can be categorized as hard capsules and soft capsules. According to different release profiles, there are also sustained-release capsules, controlled-release capsules, enteric-coated capsules, etc. The hard capsule (commonly known as capsule) refers to a capsule obtained by filling a hollow capsule with uniform powders, granules, tablets, pellets, semi-solid or liquid, or the like made of a drug substance and optionally an appropriate excipient using an appropriate preparation technology. Hard capsules can be obtained by filling hollow capsules with different forms of contents prepared by the following preparation techniques: (1) preparing the drug substance and a suitable excipient such as a diluent, glidant, disintegrating agent, or the like into uniform powders, granules, or small tablets; (2) filling with ordinary pellets, immediate-release pellets, sustained-release pellets, controlled-release pellets, or enteric-coated pellets individually or in combination, and if necessary, adding an appropriate amount of blank pellets as a filler; (3) directly filling with the drug substance powders; (4) preparing the drug substance into an inclusion, solid dispersion, microcapsules, or microspheres; or (5) filling hollow capsules with a solution, suspension, emulsion, or the like using a special encapsulating machine, and sealed the capsules if necessary. The soft capsule refers to a capsule obtained by directly sealing a certain amount of liquid drug substance in a soft capsule material, or sealing a solution, suspension, emulsion, or semi-solid prepared by dissolving or dispersing solid drug substance in a suitable excipient in a soft capsule material. It can be prepared by a dropping method or a pressing method. The soft capsule material is generally made from gelatin for capsules, glycerol, or other suitable pharmaceutical excipients alone or in combination. The sustained-release capsule refers to a capsule that slowly releases the drug from a prescribed release medium at a non-constant rate. The controlled-release capsule refers to a capsule that slowly releases the drug from a prescribed release medium at a constant rate. The enteric-coated capsule refers to a hard capsule made by filling a capsule with enteric-coated granules or pellets, or a hard or soft capsule prepared with an appropriate enteric-coating material. The enteric-coated capsule is not soluble in gastric fluid but can disintegrate in intestinal fluid to release the active ingredient.

In some embodiments, the composition is a soft capsule. The soft capsule refers to a capsule obtained by directly sealing a certain amount of liquid drug substance in a soft capsule material, or sealing a solution, suspension, emulsion, or semi-solid prepared by dissolving or dispersing solid drug substance in a suitable excipient in a soft capsule material. The soft capsule is a capsule which has a certain elasticity and softness due to the presence of a softener such as glycerol, sorbitol, maltitol, or polyethylene glycol in the capsule shell. The soft capsule can be manufactured on the basis of, for example, gelatin or starch. Gelatin-based soft capsules are commercially available from various suppliers. Depending on the mode of administration, e.g., oral or rectal administration, the soft capsules can have various shapes, which may be, for example, round, oval, elliptical, or torpedo-shaped. The soft capsule can be manufactured by a conventional process, such as the Scherer process, the Accogel process, or the dropping or blow molding process.

In some embodiments, the composition is a microencapsulated capsule. The encapsulation protects the composition from degradation until delivery to the target location, where the composition ruptures by, for example, a chemical or physical stimulus such as pressure, enzymatic activity, or physical decomposition, which can be triggered by a change in pH. Any suitable encapsulation method can be used. Exemplary encapsulation techniques include entrapment within a porous matrix, attachment or adsorption on the surface of a solid carrier, self-aggregation by flocculation or using a cross-linking agent, and mechanical accommodation behind a microporous membrane or microencapsulated capsule.

In some embodiments, the composition is made by a freeze-drying method or a spray-drying method. The freeze-drying method involves freezing a liquid pharmaceutical solution at a reduced temperature into a solid, followed by sublimation drying under vacuum to remove ice crystals, and resolution drying under vacuum after the sublimation to remove bound water. The spray-drying method is a drying method which involves preparing a solution into droplets using a micronization device, and then contacting the droplets with drying air at a relatively high temperature, so as to evaporate water. It is widely used for products with different properties in the food, pharmaceutical, and chemical industries. The spray-drying method can be an electrostatic spray-drying method, which allows the water or other solvents in the spray-dried atomized droplets to repel each other at the edge of the droplets under electrostatic interaction, and the core solid components to be retained in the center of the droplets, thereby reducing the difficulty of drying and evaporating water, i.e., the temperature required for water evaporation, and reducing the loss, degradation, or denaturation of the core active ingredients due to a high temperature.

As used herein, the composition includes a pharmaceutical composition, a health care product, or a food product.

The pharmaceutical composition provided herein can comprise an excipient. A suitable pharmaceutically acceptable excipient that can be used is, for example, a carrier, an excipient, a diluent, a lubricant, a wetting agent, an emulsifier, a suspension stabilizer, a preservative, a sweetener, or a flavor. For example, the pharmaceutically acceptable excipient is one or more of lactose, glucose, sucrose, sorbitol, mannose, starch, gum arabic, calcium phosphate, alginate, gelatin, calcium silicate, fine crystalline cellulose, polyvinylpyrrolidone, cellulose, water, syrup, methylcellulose, methylparaben, propylparaben, talc, magnesium stearate, and mineral oil.

The pharmaceutical composition provided herein can comprise a pharmaceutically acceptable excipient, diluent, or carrier. Acceptable carriers or diluents for therapeutic use are well known in the pharmaceutical field. Examples of suitable carriers include lactose, starch, dextrose, methylcellulose, magnesium stearate, mannitol, sorbitol, etc. Examples of suitable diluents include ethanol, glycerol, and water. The pharmaceutical carrier, excipient, or diluent can be selected depending on the intended route of administration and standard pharmaceutical practice. The pharmaceutical composition can comprise any suitable binder, lubricant, suspending agent, coating agent, solubilizer, or the like as or in addition to the carrier, excipient or diluent. Examples of a suitable binder include starch, gelatin, a natural sugar, and a natural or synthetic gum. The natural sugar is, for example, glucose, anhydrous lactose, free-flowing lactose, β -lactose, or corn sweetener. The natural or synthetic gum is, for example, gum arabic, tragacanth, sodium alginate, carboxymethyl cellulose, or polyethylene glycol. Examples of a suitable lubricant include sodium oleate, sodium stearate, magnesium stearate, sodium benzoate, sodium acetate, sodium chloride, or the like. A preservative, a stabilizer, a dye, or even a flavoring agent can be provided in the pharmaceutical composition. Examples of the preservative include sodium benzoate, sorbic acid, or parabens. An antioxidant or a suspending agent can also be used. An antioxidant or a suspending agent can also be used.

The composition provided herein can comprise a probiotic.

The term "probiotic" refers to an alive bacterium or yeast that is beneficial to the host's health upon consumption. It is believed that a probiotic can restore the balance of intestinal bacteria that have been disrupted by prolonged antibiotic use or gastrointestinal disorders. The probiotic includes, but is not limited to, a microorganism of the genera *Bifidobacterium, Lactobacillus, Caseobacter, Lactobacillus mucosus, Lactobacillus plantarum, Ligilactobacillus, Latilactobacillus sakei, Streptococcus, Propionibacterium, Leuconostoc, Pediococcus, Weissella, Planococcus, staphylococcus,* or *Kluyveromyces.*

Depending on the disease and patient to be treated and the route of administration, the pharmaceutical composition or pharmaceutical formulation of the present disclosure can be administered in different dosages once or multiple times a day. The dosage of administration depends on many factors, such as the severity of the disease to be treated or prevented, the gender, age, weight and individual response of the patient, the route of administration and the frequency of administration, etc. The above dosage can be administered in the form of a single dose or several divided doses, e.g., two, three, or four doses.

The actual dosage level of the main drug in the pharmaceutical formulation of the present disclosure can be varied so that the desired therapeutic response can be effectively achieved for the specific patient, composition and mode of administration. The dosage level should be selected based on the route of administration, the severity of the condition being treated, and the condition and medical history of the patient to be treated. However, it is the practice in the art to start at a level below the dosage level required for the desired therapeutic effect, and gradually increase the dosage until the desired effect is achieved.

As used herein, the term "subject" or "patient" refers to any mammal. A subject or patient "in need thereof" as described herein refers to an individual in need of treatment (or prevention) of a disease. The mammal includes human, laboratory animals (e.g., a primate, rat, or mouse), farm animals (e.g., cow, sheep, goat, or pig), and domestic pets (e.g., dog, cat, or a rodent). The subject can be human. The subject can be a non-human mammal, including, but not limited to, dog, cat, cow, horse, pig, donkey, goat, camel, mouse, rat, guinea pig, sheep, camel, monkey, gorilla, or chimpanzee. The subject or patient can be healthy or can suffer from a metabolic disease at any stage of development.

The composition of the present disclosure comprising the *Collinsella* sp. MNH 18574, a metabolite, a fermentation culture broth, a supernatant of a fermentation culture broth thereof, or any combination thereof, can be used for preventing or treating a tumor.

As used herein, the term "treating" a disease in a subject or "treating" a subject suffering or suspected of suffering from a disease refers to administering to the subject a drug therapy, such as one or more agents, thereby reducing or preventing the deterioration of at least one symptom of the disease. Therefore, in one embodiment, "treating" means *inter alia* delaying progression, accelerating remission, inducing remission, increasing remission, accelerating recovery, increasing the efficacy of an alternative therapy, decreasing the resistance to an alternative therapy, or a combination thereof.

As used herein, the term "prevention" is well-recognized in the art and, when used in connection with a condition such as local recurrence, is well known in the art. It comprises administering a treatment that reduces the development of, or delays the onset of, a symptom of a medical disorder in a subject as compared to a subject who does not receive the composition. Therefore, the prevention of a cancer includes, for example, reducing the number of detectable tumors in a patient population receiving prophylactic treatment relative to an untreated control population, and/or delaying the occurrence of detectable tumors in a treated population relative to an untreated control population, for example, by a statistically and/or clinically significant amount.

As used herein, the term "enhancing" the efficacy of a cell therapy (e.g., chimeric antigen receptor T-cell (CAR-T) therapy) refers to a result of administration of the composition of the present disclosure from the cell therapy (e.g., in the case of CAR-T, T-cell-mediated immune response, particularly against a tumor antigen), when compared to the absence of such administration. For example, a subject treated with the composition of the present disclosure and a cell therapy can exhibit greater such therapeutic effects from the cell therapy than a control subject treated with the cell therapy rather than the composition of the present disclosure.

As used herein, the term "effective amount" refers to an amount that is therapeutically or prophylactically effective for the subject being treated. The precise amount of the therapeutic composition also depends on the practitioner's judgment and is likely to be unique to each individual. The appropriate regimen for initial administration and booster administration is also variable, but is represented by the initial and subsequent administrations. The factors affecting the dosage include the physical and clinical status of the patient, the route of administration, the intended therapeutic goal (symptom relief or curing), the efficacy, stability, and toxicity of the particular therapeutic substance, or the efficacy of other treatments the subject may receive.

Excessive cell proliferation can lead to the development of a tumor or neoplasm. Tumor cells are considered to be cancerous when they have the ability to invade surrounding tissues, for example, by breaking down and entering the bloodstream or lymphatic system, or forming secondary tumors elsewhere in the body. Therefore, as used herein, the term "tumor" includes both benign tumors and malignant tumors (i.e., cancers). In some instances, the terms "tumor" and "cancer" are used interchangeably to refer to the presence of cells having characteristics typical of cancer cells, which include, but are not limited to, uncontrolled proliferation, immortalization, metastatic potential, rapid growth and proliferation rate, as well as certain characteristic morphological features.

Without wishing to be bound by theory, it is believed that the composition of the present disclosure can inhibit the growth of the tumor by immunomodulating macrophages, primary PBMCs, and/or monocyte-derived dendritic cells.

Recent studies have shown that the gut microbes in cancer patients have a significant influence on the efficacy of tumor immunotherapy, and that modulation and utilization of gut microbes have become key components of tumor immunotherapy.

It has been demonstrated that there are multiple mechanisms of direct interaction between gut microbes and immune cells, and that dendritic cells and macrophages in intestinal lymphoid tissues are key target cells for immunomodulatory microorganisms. Particularly in the small intestine, the large intestinal surface area and the thin and diffuse mucous layer allow microorganisms to come into close contact with immune cells. Different specific strains in the microflora can inhibit or activate immune responses throughout the body.

Macrophages are a heterogeneous population of myeloid cells in the natural immune system, which are involved in a variety of physiological and pathological processes. During inflammation or infection in an organism, blood monocytes accumulate in tissues and differentiate into macrophages. Macrophages are highly plastic and have two main polarized states, i.e., classically activated type 1 (M1) and alternatively activated type 2 (M2). M1 macrophages are macrophages that can produce pro-inflammatory cytokines, known as classic macrophages, and often occur after injury and infection. Classical activation of macrophages *in vitro* often occurs through the bacterial cell wall (e.g., LPS) and TNF$\alpha$ or IFN$\gamma$. M1 macrophages are characterized by the secretion of pro-inflammatory factors such as TNF$\alpha$, IL-1$\beta$, IL-6, IL-12, and IL-8, which play an important role early in the inflammation. Polarization of M2 macrophage can be induced by different stimuli, mainly including IL-4 and/or IL-13. Macrophages activated by IL-4 and IL-13 secrete anti-inflammatory cytokines, such as IL-10, CCL18, and CCL22, which play a role in inhibiting inflammatory responses and tissue repair.

M1 macrophages are cytotoxic to both pathogens and tumor cells. Their anti-tumor activity is related to their ability to secrete reactive nitroxide, reactive oxygen species, and pro-inflammatory cytokines. M2 macrophages promote the growth and survival of tumor cells by secreting many growth factors such as EGF, TGF-$\beta$, VEGF, or the like. Therefore, detection of the expression of macrophage-associated pro-inflammatory and anti-inflammatory factors induced by different strains can be used as a means to judge whether the strain has a possible anti-tumor profile.

The composition of the present disclosure can also achieve an anti-tumor effect by inhibiting a tumor through a short-chain fatty acid or a short-chain fatty acid salt. The short-chain fatty acid is a lipid consisting of 2 to 6 carbon atoms, including acetate, propionate, butyrate, etc., which are mainly produced through fermentation of dietary fibers by gut microbes, and have been newly shown to have potential anticancer effects in a variety of extraintestinal cancers such as bladder, breast, stomach, liver, lung, pancreatic, and prostate cancers (doi: 10.1016/j.biopha.2021.111619). SCFA can be specifically recognized by G protein-coupled receptor (GPCR) on the surface of cancer cells. In addition, due to its small molecular weight, SCFA can enter the nucleus of cancer cells, inhibit the activity of histone deacetylase (HDAC), and then upregulate histone acetylation and crotonylation, thereby exerting a direct anticancer effect. SCFA can also play an indirect anticancer role by regulating immune cells. SCFA is produced by fermentation of dietary fibers by intestinal bacteria, which can not only act on the intestinal tract, but also reach the liver and the whole body through the portal vein system to play an anticancer role. After being specifically recognized by the GPCR on the surface of cancer cells, SCFA exerts the effects of promoting cancer cell apoptosis, arresting cell cycle, and inhibiting cancer cell metastasis by increasing the secretion of TNF-$\alpha$, upregulating the expression of Fas-L, reducing the expression of Bcl-2, downregulating the activities of CDK and Cyclin, or inhibiting the MAPK/ERK pathway. After entering the nucleus of cancer cells, SCFA exerts the effects of promoting cancer cell apoptosis, arresting cell cycle, and inhibiting cancer cell metastasis by inhibiting HDAC, increasing the secretion of TNF-$\alpha$, upregulating the expression of Fas-L, reducing oxygen consumption or lactic acid metabolism, downregulating the activity of Cyclin, upregulating the expression of CDK inhibitor p21 gene, or inhibiting the Akt/ERK pathway. SCFA can enhance the secretion of IgA and IgG by B cells, the secretion of IFN-$\gamma$ by T cells, the expression of MICA/B on the surface of cancer cells, which in turn increases the recognition ability of NK cells, and the production of ROS by macrophages (doi: 10.12354/j.issn.1000-8179.2022.20211821). Therefore, it is expected that the microbial agent or drug of the present disclosure can inhibit a tumor via SCFA.

The composition of the present disclosure comprising the *Collinsella* sp. MNH 18574, a metabolite, a fermentation culture broth, a supernatant of a fermentation culture broth thereof, or any combination thereof, can be used for preventing or treating a metabolic disorder, including, but not limited to, obesity, an obesity-related disease, diabetes, a diabetes-related disease, a liver function disorder, a liver and kidney function disorder, inflammation, a metabolic disorder caused by obesity, a cardiovascular or cerebrovascular disease, dyslipidemia, abnormal lipid metabolism, hypercholesterolemia, or atherosclerosis.

The term "obesity" refers to a certain degree of marked overweight with a thick fat layer, which is a state caused by excessive accumulation of body fat, especially triglycerides, and abnormal or excessive fat accumulation that poses a risk to the health. Excessive body fat accumulation as a result of excessive food intake or altered metabolism results in excessive weight gain and causes a pathological or physiological change or latency. A body mass index (BMI) of over 25 is considered overweight and over 30 is considered obese. Obesity will increase the risk of many physical and mental diseases. It is mainly associated with metabolic syndrome, including a combination of diseases such as type 2 diabetes, hypertension, hypercholesterolemia, hypertriglyceridemia, etc. Generally, the effects of obesity on health fall into two broad categories: diseases attributable to increased body fat (e.g., osteoarthritis, obstructive sleep apnea, etc.) and diseases with an increased number of adipocytes (e.g., diabetes mellitus, dyslipidemia, cancer, cardiovascular disease, non-alcoholic fatty liver disease, non-alcoholic steatohepatitis, etc.). The term "obesity-related disease" includes obesity, overeating, binge eating, bulimia, hypertension, diabetes mellitus, elevated plasma insulin concentration, insulin resistance, hyperlipidemia, metabolic syndrome, insulin resistance syndrome, obesity-related gastroesophageal reflux disease, atherosclerosis, hypercholesterolemia, hyperuricemia, lower back pain, cardiomegaly and left ventricular hypertrophy, lipodystrophia, non-alcoholic steatohepatitis, cardiovascular disease, polycystic ovary syndrome, etc.

The term "diabetes mellitus" includes type I diabetes (T1D), type II diabetes (T2D), and gestational diabetes mellitus (GDM). Type I diabetes is a type of diabetes mellitus caused by autoimmune impairment or an idiopathic cause, characterized by absolute destruction of pancreatic islet functions, which mostly occurs in children and adolescents, and must be treated with insulin in order to obtain a satisfactory outcome, or it will be life-threatening. Type II diabetes is a multifactorial syndrome characterized by abnormal carbohydrate/fat metabolism, usually including hyperglycemia, hypertension, and abnormal cholesterol. Type II diabetes is caused by ineffective role of insulin (low binding to the receptor). Therefore, it is important to test not only fasting blood glucose, but also 2-hour postprandial blood glucose, and especially to perform a pancreatic islet function test. There are two types of diabetes during pregnancy: diabetes diagnosed before pregnancy, called "diabetes with pregnancy"; and diabetes that occurs or is diagnosed only during pregnancy, with normal glucose metabolism or potentially impaired glucose tolerance before pregnancy, also known as "gestational diabetes mellitus (GDM)". More than 80% of pregnant women with diabetes suffer from GDM.

The term "diabetes-related disease" includes at least one of type I diabetes (T1D), type II diabetes (T2D), gestational diabetes mellitus (GDM), insulin resistance syndrome, glucose intolerance, hyperlipidemia, a complication of diabetic nephropathy, diabetic neuropathy, diabetic ophthalmopathy, a cardiovascular disease, and diabetic foot.

The term "insulin resistance" refers to a decrease in the efficiency of insulin in promoting glucose uptake and utilization due to various reasons, and compensatory secretion of excessive insulin causing hyperinsulinemia in order to maintain the stability of blood glucose. Insulin resistance is prone to lead to metabolic syndrome and type II diabetes.

The term "liver function disorder" refers to a disease associated with abnormal liver function or impaired liver function. Alanine aminotransferase (ALT) and aspartate aminotransferase (AST) are sensitive markers for liver function disorders. Blood ALT and/or AST levels are markedly elevated in case of abnormal liver function (e.g., injury, non-alcoholic fatty liver disease (NAFLD), or non-alcoholic steatohepatitis (NASH)). In general, ALT is more sensitive than AST to reflect acute liver injury. Persistent elevation of ALT suggests chronic liver injury. AST is significantly elevated and can exceed ALT in case of chronic hepatitis, cirrhosis, liver cancer, etc. The AST level indicates the chronicity, extent, and severity of a liver disorder, or even the prognosis of a chronic liver disease. The most common liver diseases with elevated ALT and AST levels include acute viral hepatitis (hepatitis A to E), Epstein-Barr virus infection, cytomegalovirus infection, chronic hepatitis B and C, autoimmune liver disease, alcoholic liver disease (ALD), non-alcoholic fatty liver disease (NAFLD and NASH), drug-induced/toxic liver injury, cirrhosis, liver cancer, hepatolenticular degeneration, $\alpha$1-antitrypsin deficiency, hemochromatosis, etc.

The term "disease related to liver function impairment" includes at least one of fatty liver, non-alcoholic fatty liver disease, non-alcoholic steatohepatitis, liver fibrosis, cirrhosis, and liver cancer.

The term "non-alcoholic fatty liver disease (NAFLD)" refers to accumulation of excessive fat in the liver in the form of triglycerides (steatosis). There are also some NAFLD patients who suffer from hepatocellular damage and inflammation in addition to excessive fat (steatohepatitis), i.e., non-alcoholic steatohepatitis (NASH). NASH is widely recognized as a hepatic manifestation of metabolic syndrome such as type II diabetes, insulin resistance, central obesity, hyperlipidemia (low high-density lipoprotein cholesterol, hypertriglyceridemia), and hypertension. In addition, intrahepatic fat accumulation is an important aspect of the development of non-alcoholic fatty liver disease (NAFLD or NASH). Therefore, a reduction in ALT and AST levels and a reduction in liver weight after drug intervention can indicate some therapeutic or ameliorative effects of the drug.

The term "liver and kidney function disorder" refers to functional acute renal failure or decompensated liver cirrhosis that occurs in a severe liver disease, which can result in hepatorenal syndrome due to insufficient effective circulating blood volume, reduced prostaglandins, or the like.

The composition of the present disclosure comprising the *Collinsella* sp. MNH 18574, a metabolite, a fermentation culture broth, a supernatant of a fermentation culture broth thereof, or any combination thereof, can be used for weight management in a subject.

The term "weight management" refers to at least one of reducing body weight, maintaining body weight, maintaining body weight loss (also referred to herein as weight maintenance), controlling body weight gain, reducing body mass index (BMI), maintaining BMI, maintaining BMI reduction, and controlling BMI increase in a subject. In some embodiments, the subject is overweight or obese. In some embodiments, the subject suffers from at least one weight-related disorder. In some embodiments, the at least one weight-related disorder is selected from metabolic diseases or disorders, metabolic syndrome, and cardiovascular diseases. In some embodiments, the at least one weight-related disorder is selected from hypertension, dyslipidemia, and type 2 diabetes. In some embodiments, at least one symptom of the at least one weight-related disorder is ameliorated. For example, weight management for an overweight or obese individual can mean weight loss with the goal of maintaining the body weight in a healthier range. In addition, for example, weight management for an overweight or obese individual can include reducing body fat (reducing BMI) or waist circumference with or without weight loss. Maintaining weight loss (weight maintenance) includes preventing, reducing, or controlling body weight gain following weight loss. It is well known that body weight gain usually occurs after body weight loss. For example, body weight loss can occur as a result of dieting, exercise, illness, medication, surgery, or any combination thereof. However, individuals who have lost weight tend to regain some or all of the weight they have lost. Accordingly, weight maintenance for an individual who has lost weight can include preventing body weight gain after body weight loss, reducing the amount of body weight gain after body weight loss, controlling body weight gain after body weight loss, or slowing the rate of body weight gain after body weight loss. As used herein, the term "weight management in an individual in need thereof" refers to the judgment made by a healthcare practitioner that the individual is in need of, or would benefit from, the weight management treatment. The judgment is based on a variety of factors in the field of expertise of the healthcare practitioner, but includes the situation where the individual suffers from a disorder that can be treated by the method as disclosed herein. In some embodiments, the weight management comprises reducing appetite. In some embodiments, the weight management comprises reducing sensation of hunger.

Examples and accompanying drawings are provided below to help understand the present disclosure. It should be understood, however, that these Examples and drawings are intended to illustrate the present disclosure only, but do not constitute any limitation. The actual scope of protection of the present disclosure is set forth in the claims. It should be understood that any modifications and changes can be made without departing from the spirit of the present disclosure.

The liquid MM01 culture medium involved in the Examples of the present disclosure comprises: peptone 5 g/L, trypsinized casein 5 g/L, yeast powder 10 g/L, beef extract 5 g/L, glucose 5 g/L, $K_2HPO_4$ 2 g/L, sodium acetate 2 g/L, Tween 80 1 mL/L, hemoglobin 5 mg/L, L-cysteine hydrochloride 0.5 g/L, vitamin K1 1 uL/L, and an inorganic salt solution 8 ml/L, which comprises 0.25 g of calcium chloride, 1 g of dipotassium hydrogen phosphate, 1 g of potassium dihydrogen phosphate, 0.5 g of magnesium sulfate, 10 g of sodium bicarbonate, and 2 g of sodium chloride per L of the inorganic salt solution.

The solid MM01 culture medium involved in the Examples of the present disclosure comprises: peptone 5 g/L, trypsinized casein 5 g/L, yeast powder 10 g/L, beef extract 5 g/L, glucose 5 g/L, $K_2HPO_4$ 2 g/L, sodium acetate 2 g/L, Tween 80 1 mL/L, hemoglobin 5 mg/L, L-cysteine hydrochloride 0.5 g/L, vitamin K1 1 uL/L, an inorganic salt solution 8 ml/L, which comprises 0.25 g of calcium chloride, 1 g of dipotassium hydrogen phosphate, 1 g of potassium dihydrogen phosphate, 0.5 g of magnesium sulfate, 10 g of sodium bicarbonate, and 2 g of sodium chloride per L of the inorganic salt solution, and agar 15 g/L.

The AC liquid medium involved in the Examples of the present disclosure comprises (per liter): peptone, 20 g; glucose, 5 g; yeast extract, 3 g; beef extract powder, 3 g; and vitamin C, 0.2 g; pH 7.0.

The anaerobic blood plate (purchased from Huankai Microbial) involved in the Examples of the present disclosure has the following formula (per liter): pancreatic digest of casein 10.0 g, cardiac pancreatic digest 3.0 g, corn starch 1.0 g, pepsin digest of meat 5.0 g, yeast extract powder 5.0 g, sodium chloride 5.0 g, agar 15.0 g, sterile defibrinated goat blood 50-100 mL, and distilled water 1000 mL; final pH $7.3\pm0.2$.

The tryptic soytone broth (TSB) liquid medium involved in the Examples of the present disclosure comprises (per liter): tryptic soytone 17.0 g, papain hydrolysate of soybean 3.0 g, dipotassium hydrogen phosphate 2.5 g, sodium chloride 5.0 g, and glucose 2.5 g; pH $7.3\pm0.2$.

The MPYG culture medium involved in the Examples of the present disclosure was prepared according to the PYG MEDIUM (modified) formula from the Leibniz Institute DSMZ-German Collection of Microorganisms and Cell Cultures

(https://www.dsmz.de/microorganisms/medium/pdf/DSMZ_Medium104.pdf).

The culture media as described above can be prepared using conventional methods of formulation and sterilization.

In the Examples of the present disclosure, the solvent (also known as PBS-Cys (gly)) was prepared by evenly mixing PBS-Cys (a phosphate buffer containing 0.05% cysteine hydrochloride) with 100% glycerol in a ratio of 3:1.

In the Examples of the present disclosure, the Tumor Growth Inhibition rate (TGI) was calculated according to the following equation:

Tumor Growth Inhibition rate = (Average volume in the control group - Volume in the experimental group) / Average volume in the control group $\times$ 100%.

All data were expressed as mean $\pm$ SD, and plotted and statistically analyzed using the GraphPad Prism 8.0.2 software. For pairwise comparisons, t-test (Student's t test) was used. Two-way analyses were performed by 2-way ANOVA with Sidak multiple comparison. The significance of difference was indicated by *: * $p < 0.05$, ** $p < 0.01$, *** $p < 0.001$, **** $p < 0.0001$.

In the Examples of the present disclosure, the response rate was calculated according to the following equation:

$$\text{Response rate} = \text{Number of mice with response} / \text{Number of surviving mice in the group} \times 100\%.$$

Response: an average tumor volume greater than 40% tumor growth inhibition rate indicates non-response, and an average tumor volume less than or equal to 40% tumor growth inhibition rate indicates response. 40% tumor growth inhibition rate = Average tumor volume in the negative control group $\times$ (1 - 40%) $\times$ 100%.

Example 1: **Isolation and identification of the strain**

### 1.1 Isolation of the strain

The intestinal strain MNH 18574 involved in the present disclosure was isolated from a stool sample from a healthy volunteer in Tibet.

2-5 g of fresh stool was collected by the donor and placed into a sample collection and storage tube. After being homogenized by shaking, the processed stool sample was placed in an ice box and sent to the laboratory within 24 hours for strain isolation.

Specifically, the fresh stool sample was placed in an anaerobic operation station (Don Whitley Scientific H35), and mixed well by shaking on a vortex shaker for 1 min. 1 mL of the sample was pipetted into 9 mL of physiological saline, mixed well to afford a $10^{-1}$ diluted solution, and then serially diluted to $10^{-6}$ dilution for later use. The $10^{-6}$ diluted solution was dropped onto an isolation medium anaerobic blood agar plate (Huankai Microbial) in an amount of 100 $\mu$L/plate, and spread evenly. After the plate surface was dried, the plate was inverted and incubated at 37 °C for 3-5 days. The growth status of the strain was observed, and a single colony was picked up with a sterilized toothpick for strain purification. The purified strain was anaerobically cultured on an anaerobic blood agar plate at 37 °C. The pure culture strain was stored in 20% (W/V) glycerol at -86 °C. The growth status of the strain was observed, and a single colony was picked up with a sterilized toothpick for strain purification. The strain was named MNH 18574. The purified strain was anaerobically cultured at 37 °C.

The purified and cultured strain was prepared into a 20% glycerol/water-bacterial solution and stored at -86 °C.

The strain was identified using a method based on 16S rRNA gene sequence identification.

### 1.2 Profile of the strain

### 1.2.1 Deposit of the strain

The strain MNH 18574 was deposited on August 4, 2022 with the Guangdong Microbial Culture Collection Center with a deposit name of *Collinsella* sp. MNH 18574 and a deposit number of GDMCC 62667. The deposit address is Guangdong Institute of Microbiology, 5th Floor, No.59 Building, No. 100 Xianlie Zhong Road, Guangzhou. The proposed taxonomic name is *Collinsella* sp.

### 1.2.2 Morphological features of the strain

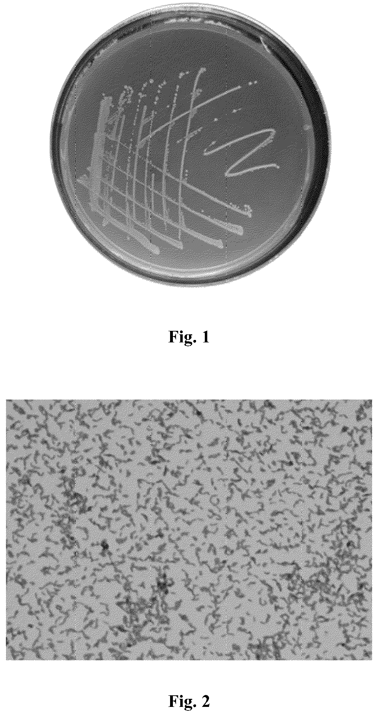

After the strain MNH 18574 was inoculated onto an MYPG solid medium plate and anaerobically cultured at 37 °C for 48 hours, visible colonies were formed on the plate. The colonies were round, milky white, opaque, and about 2-3 mm in diameter, with regular and smooth edges, and without secretion formed around the colonies, as shown in Fig. 1.

The results from conventional Gram staining are shown in Fig. 2, indicating that the strain MNH 18574 isolated in the present disclosure is Gram-negative.

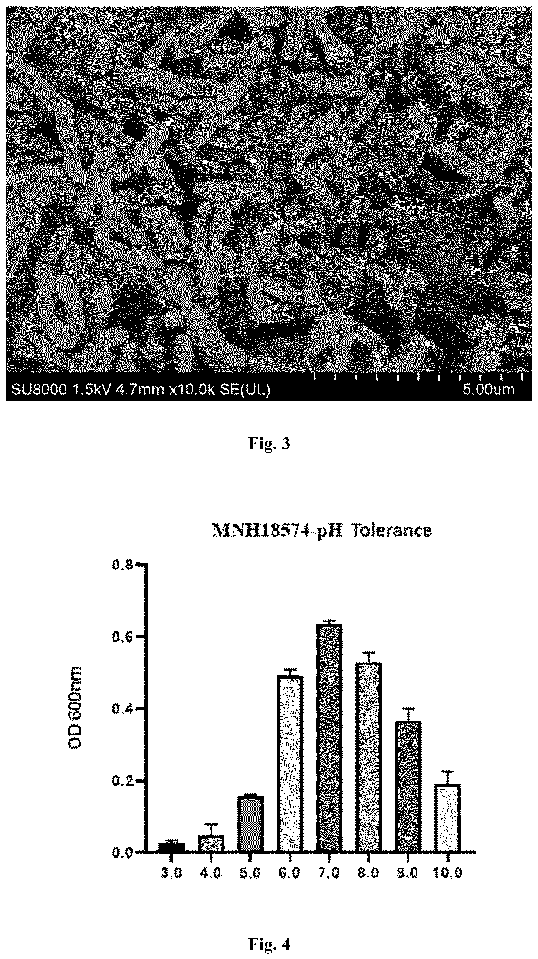

Morphological observation under electron microscope showed that the strain had no spores, no flagella, was non-motile, short rod-shaped, and had a size of about 5 $\times$ 10 $\mu$m (Fig. 3).

### 1.2.3 Physiological and biochemical properties of the strain

### 1.2.3.1 pH tolerance, NaCl tolerance, and bile salt tolerance of the strain

(1) pH tolerance and NaCl tolerance

The strain was inoculated into MM01 culture medium, sterilized, centrifuged, and then splitted into different centrifuge tubes for later use. The bacterial solution in the centrifuge tubes was inoculated into MM01 culture media at different pH values, MM01 culture media with different concentrations of NaCl, and MM01 culture media with different concentrations of sodium cholate. Three replicates were set up for each concentration. The bacterial solution was mixed well after inoculation. Then, 200 $\mu$L of the bacterial solution was pipetted into a 96-well microtiter plate, and measured for the absorbance at 600 nm.

(2) Bile salt tolerance

MM01 culture medium was dispensed into 100 mL reagent bottles, 100 mL per bottle. 0.10, 0.15, 0.20, 0.20, 0.30, and 0.40 g of sodium cholate were added to prepare MM01 culture media containing sodium cholate at a concentration of 0%, 0.10%, 0.15%, 0.20%, 0.25%, 0.30%, and 0.40%, respectively. After sterilization and cooling down to room temperature, the media were dispensed into 10 mL centrifuge tubes, 6 mL per tube. Three replicates were set up for each concentration.

0.6 mL of the first generation of bacterial solution was pipetted or a single colony was scraped into 30 mL of MM01 culture medium, and cultured under anaerobic conditions at 37 °C for 14-24 h, to afford the second generation of bacterial solution. 120 μL of the second generation of bacterial solution was inoculated into 6 mL of MM01 culture media containing different concentrations of bile salts using a micropipette. At 24 h after inoculation, the bacterial solution was mixed well with a micropipette. 200 μL of the bacterial solution was pipetted into a 96-well microtiter plate, and measured for the absorbance at 600 nm.

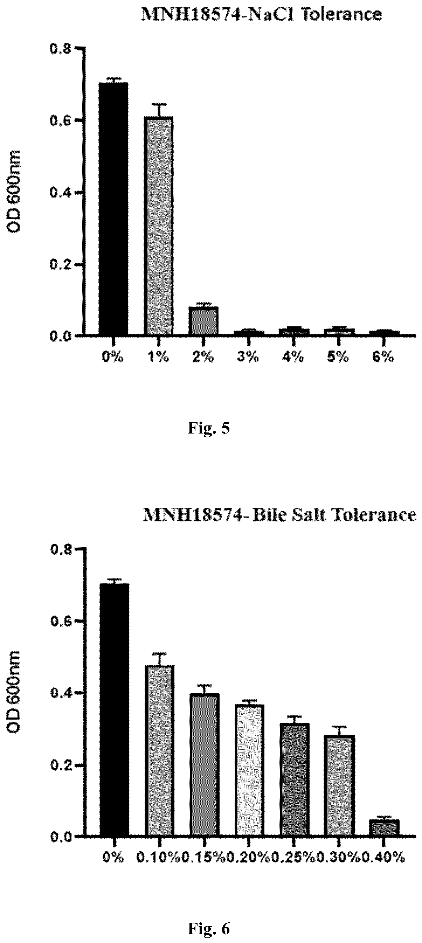

The results are shown in Figs. 4-6. The strain MNH 18574 grew at a temperature ranging from 30 to 42 °C, with an optimal growth temperature of 37 °C. It could grow at pH 5.0-10.0, with an optimal growth pH of 6.0-8.0. It could tolerate up to 1% NaCl. The strain MNH 18574 could survive and grow at a bile salt concentration in the range of 0-0.30%, and could not grow at a bile salt concentration greater than or equal to 0.40%. The strain MNH 18574 could not grow under aerobic conditions, but grew well under anaerobic conditions, and thus is an obligate anaerobic bacterium.

## 1.2.3.2 Physiological and biochemical properties of the strain MNH 18574 by API 20A test

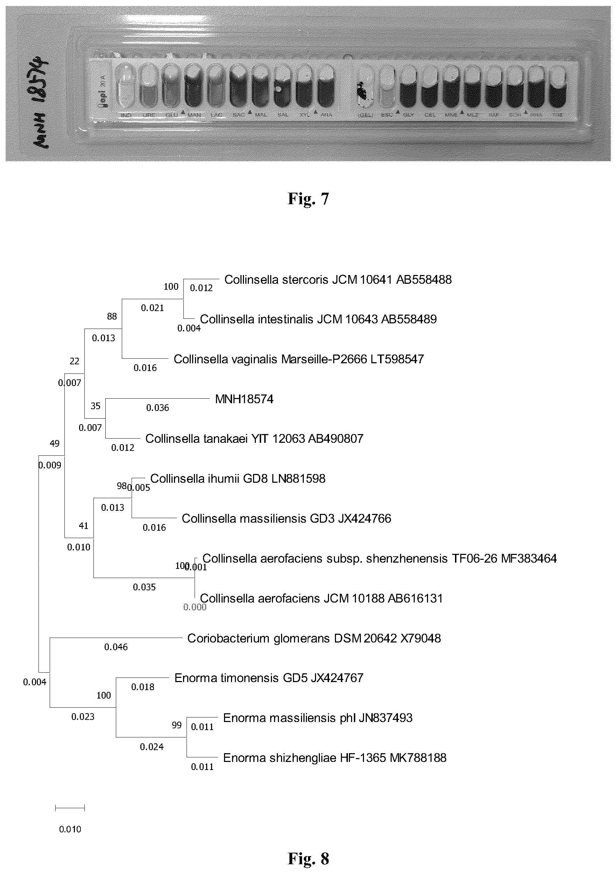

API reagent strip (BioMérieux). The strain was anaerobically cultured at 37 °C. The test results are shown in Table 1 and Fig. 7.

Table 1. Results from API 20A test of the strain MNH 18574

| Test item | Test result | Test item | Test result |
|---|---|---|---|
| IND | Negative | ESC | Negative |
| URE | Negative | GLY | Non-acid producing |
| GLU | Acid-producing | CEL | Non-acid producing |
| MAN | Non-acid producing | MNE | Non-acid producing |
| LAC | Acid-producing | MLZ | Non-acid producing |
| SAC | Non-acid producing | RAF | Non-acid producing |
| MAL | Non-acid producing | SOR | Non-acid producing |
| SAL | Non-acid producing | RHA | Non-acid producing |
| XYL | Non-acid producing | TRE | Non-acid producing |
| ARA | Non-acid producing | GEL | Negative |

## 1.2.3.3 Antibiotic sensitivity test of the strain MNH 18574

An antibiotic sensitivity test was carried out on the strain MNH 18574 by a disk diffusion method. The test results are shown in Table 2. The strain MNH 18574 was sensitive to antibiotics such as chloramphenicol and penicillin, and resistant to antibiotics such as gentamicin, erythromycin, tetracycline, ciprofloxacin, trimethoprim-sulfamethoxazole, ampicillin, lincomycin, and ceftriaxone.

Table 2. Results from Antibiotic sensitivity test of the strain MNH 18574

| Antibiotic | Diameter of inhibition zone (mm) |
|---|---|
| Gentamicin (GEN) | 0 |
| Erythromycin (ERM) | 0 |
| Chloramphenicol (CLM) | 27.64 |
| Tetracycline (TET) | 0 |

(continued)

| Antibiotic | Diameter of inhibition zone (mm) |
|---|---|
| Penicillin (PEN) | 24.51 |
| Ciprofloxacin (CFX) | 0 |
| Trimethoprim-sulfamethoxazole (T/S) | 0 |
| Ampicillin (AMP) | 0 |
| Lincomycin (LIN) | 0 |
| Ceftriaxone (CTR) | 0 |

**1.3 Identification of the strain MNH 18574**

**1.3.1 Identification of 16S rRNA of the strain MNH 18574**

Genomic DNA was extracted from the strain MNH 18574. 16S rRNA amplification was conducted using the extracted genomic DNA as a template. The amplification primers were 1492R (5'-AGAGTTTGATCATGGCTCAG-3') (SEQ ID No: 2) and 27F (5'-TAGGGTTACCTTGTTACGACTT-3') (SEQ ID No: 3). The amplified PCR product was purified and then sequenced by ABI3730XL to obtain a 16S rRNA nucleotide sequence of 1346 bp in length as set forth in SEQ ID No: 1.

CAGTCGTACCAACGAGTCCATCGTAAGGAGGGAAGCGAGTGGCGAACGGCTGAGTA
ACACGTGACCAACCTGCCCGGCTCTCGGGGATAGCCGCGGGAAACCGCGGGTAATA
CCCGGCGAACCCGCCCGGACGCATGTCCGGGCGGGCGAAGCTTTCGCGGAGCCGGA
TGGGGTCGCGGCCCATCAGGTAGACGGCGGGGTGACGGCCCACCGTGCCTACAACG
GGTAGCCGGGTTGAGAGACCGACCGGCCAGATTGGGACTGAGACACGGCCCAGACT
CCTACGGGAGGCAGCAGTGGGGAATCTTGCGCAATGGGGGCAACCCTGACGCAGCG
ACGCCGCGTGCGGGACGAAGGCCTTCGGGTCGTAAACCGCTTTCAGCAGGGACGAG
TCAAGACGGTACCTGCAGAAGAAGCCCCGGCTAACTACGTGCCAGCAGCCGCGGTA
ATACGTAGGGGGCGAGCGTTATCCGGATTCATTGGGCGTAAAGCGCGCGTAGGCGG
CCGCGTAGGCGGGGGGTCAAATCCCGGGGCTCAACCCCGGTCCGCCCCCCGAACCCC
GCGGCTCGGGTCCGGTAGGGGAGGGTGGAATTCCCGGTGTAGCGGTGGAATGCGCA
GATATCGGGAGGAACACCGGTGGCGAAGGCGGCCCTCTGGGCCGAGACCGACGCTG
AGGCGCGAAAGCTGGGGGAGCGAACAGGATTAGATACCCTGGTAGTCCCAGCCGTA
AACGATGGACGCTAGGTGTGGGGGGGCGATCCCCCCGTGCCGCAGCCAACGCATTA
AGCGTCCCGCCTGGGGAGTACGGCCGCAAGGCTAAAACTCAAAGGAATTGACGGGG
GCCCGCACAAGCAGCGGAGCATGTGGCTTAATTCGAAGCAACGCGAAGAACCTTAC
CAGGGCTTGACATGCTAGTGAAGCCGGGGAGACCCGGTGGCCGAGAGGAGCTAGCA
CAGGTGGTGCATGGCTGTCGTCAGCTCGTGTCGTGAGATGTTGGGTTAAGTCCCGCA
ACGAGCGCAACCCCCGCCGCATGTTGCCAGCAGGTGAGGCTGGGCACCCATGCGGG
ACCGCCGGCGCCAAGCCGGAGGAGGGCGGGGACGACGTCAAGTCATCATGCCCCTT
ATGCCCTGGGCTGCACACGTGCTACAATGGCCGGTACAGCGGGATGCGATGGCGCG
AGCCGGAGCGGATCCCTCAAAGCCGGCCCCAGTTCGGATTGGGGGCTGCAACCCGC
CCCCATGAAGTCGGAGTTGCTAGTAATCGCGGATCAGCATGCCGCGGTGAATGCGTT
CCCGGGCCTTGTACACACCGCCCGTCACACCACCCGAGTCGTCTGCACCCGAAGC
(SEQ ID No: 1).

The sequence was compared against the NCBI 16S ribosomal RNA sequences database. It was tentatively concluded that the strain belonged to the genus *Collinsella.* The closest species was *Collinsella tanakaei,* with a similarity of 96.75%. The species taxonomic information of this strain was preliminarily determined. That is, it was tentatively determined that MNH18574 belonged to the genus *Collinsella.* According to Kim, *et al.* (2014), through the statistical analysis of thousands of genomic and 16S rRNA gene sequences, it has been found that when the similarity between the 16S rRNA gene sequences of two strains is less than 98.65%, they can be judged to belong to different species. According to this judgment criterion, the experimental strain MNH 18574 of the present disclosure represents a new species of the genus *Collinsella.*

In order to further determine the genome-wide similarity between MNH18574 of the present disclosure and other members of *Collinsella,* the genome of the original strain MNH18574 as isolated was further prepared, sequenced, assembled, and analyzed. The genome of the original strain MNH18574 was fragmented by ultrasonication with a fragmentation length range of ~350 bp, and then an Illumina sequencing library was constructed using a standard DNA library construction kit (NEB Ultra™). The constructed sequencing library was subjected to paired-end 150 bp sequencing using NovaSeq (Illumina).

The raw data of genome sequencing were filtered using fastp (version: 0.20.0). The filtered raw data were subjected to genome assembly using SPAdes (version: v3.14.0), and genomic genes were subjected to genome gene prediction and analysis using the prokaryotic analysis software genome annotation process prokka (version: 1.14.5). The genomic information of all strains under the genus *Collinsella* was downloaded from NCBI. The whole genome sequence of MNH18574 was compared to those of other strains and species of *Collinsella* by assessing the average nucleotide identity (ANI) and alignment fraction (AF) to these reference organisms, as shown in Table 3, so as to determine the degree of genome correlation to the previously identified species of the genus *Collinsella.* The comparison results showed that the type strain with the highest genome similarity was *Collinsella phocaeensis,* with an average nucleotide identity (ANI) of up to 85.41% and a gene coverage of 64.45%. The strain could be clearly differentiated from the known species *Collinsella intestinalis, Collinsella stercoris, Collinsella aerofaciens, Collinsella tanakaei, Collinsella bouchesdurho nensis, Collinsella ihuae, Collinsella phocaeensis, Collinsella vaginalis,* and *Collinsella provencensis* in terms of genotypic characteristics.

Further, the *Collinsella* sp. of the present disclosure was Gram-negative, whereas the known species *Collinsella massiliensis, Collinsella aerofaciens, Collinsella acetigenes, Collinsella avium, Collinsella bouchesdurhonensis,* and *Collinsella ihumii* were Gram-positive.

Therefore, several physiological, biochemical, and genotypic characteristics of MNH18574 were significantly different from those of the existing strains of other species under the genus *Collinsella,* and MNH18574 can be recognized as a new species under the genus *Collinsella.*

Table 3. Results from comparison between the strain MNH18574 and *Collinsella bacteria*

| | | Ref | ANI | AF |
|---|---|---|---|---|
| MNH18574.fas | *Collinsella intestinalis DSM13280* | GCF_000156175.1 | 80.9353 | 0.466158673 |
| MNH18574.fas | *Collinsella stercoris DSM 13279* | GCF_000156215.1 | 80.3787 | 0.523083819 |
| MNH18574.fas | *Collinsella aerofaciens ATCC 25986* | GCF_000169035.1 | 78.1866 | 0.448677723 |
| MNH18574.fas | *Collinsella tanakaei YIT 12063* | GCF_000225705.1 | 81.2267 | 0.556701031 |
| MNH18574.fas | *Collinsella sp. 4JJ7FAA* | GCF_000763055.1 | 78.544 | 0.440609592 |
| MNH18574.fas | *Collinsella sp. MS5* | GCF_000821265.1 | 77.6683 | 0.385029135 |
| MNH18574.fas | *Collinsella aerofaciens* | GCF_001405375.1 | 78.4166 | 0.439264904 |
| MNH18574.fas | *Collinsella aerofaciens* | GCF_001406575.1 | 78.4172 | 0.426714478 |
| MNH18574.fas | *Collinsella sp. An7* | GCF_002159335.1 | 78.0026 | 0.474675034 |
| MNH18574.fas | *Collinsella sp. An271* | GCF_002159695.1 | 78.8396 | 0.54908113 |
| MNH18574.fas | *Collinsella sp. An268* | GCF_002159765.1 | 78.3746 | 0.499327656 |
| MNH18574.fas | *Collinsella sp. An2* | GCF_002160065.1 | 78.0314 | 0.493500672 |
| MNH18574.fas | *Collinsella sp. An307* | GCF_002161805.1 | 77.999 | 0.46884805 |
| MNH18574.fas | *Collinsella sp. TF06-26* | GCF_002232035.1 | 78.2917 | 0.448677723 |
| MNH18574.fas | Collinsella sp TM09- 1 0AT[r] | GCF_003436275.1 | 78.4448 | 0.454952936 |
| MNH18574.fas | Collinsella sp TM0 5- 3 7[r] | GCF_003436455.1 | 78.2331 | 0.445091887 |
| MNH18574.fas | *Collinsella sp. TF12-2AT* | GCF_003436735.1 | 78.3158 | 0.452711788 |

(continued)

|  |  | Ref | ANI | AF |
|---|---|---|---|---|
| MNH18574.fas | Collinsella sp TF1 1-5AC' | GCF_003436775.1 | 78.4501 | 0.459435231 |
| MNH18574.fas | Collinsella sp. TF09-1AT | GCF_003436895.1 | 78.6464 | 0.437471986 |
| MNH18574.fas | Collinsella tanakaei | GCF_003436995.1 | 81.1442 | 0.565665621 |
| MNH18574.fas | Collinsella sp. TF07-1 | GCF_003437235.1 | 78.2524 | 0.448229494 |
| MNH18574.fas | Collinsella sp. TF06-6AC | GCF_003437265.1 | 78.2728 | 0.446884805 |
| MNH18574.fas | Collinsella sp. OM07-12 | GCF_003438275.1 | 78.35 | 0.447333035 |
| MNH18574.fas | Collinsella sp. TF05-9AC | GCF_003438495.1 | 78.2482 | 0.445988346 |
| MNH18574.fas | Collinsella sp OM06-18AC | GCF_003438785.1 | 78.2289 | 0.450470641 |
| MNH18574.fas | Collinsella sp. AF28-5AC | GCF_003457875.1 | 78.2381 | 0.452263559 |
| MNH18574.fas | Collinsella sp. AF25-2LB | GCF_003458415.1 | 78.3488 | 0.436127297 |
| MNH18574.fas | Collinsella sp AF23-3LB | GCF_003458685.1 | 78.3331 | 0.450470641 |
| MNH18574.fas | Collinsella sp AF23-2 | GCF_003459245.1 | 78.3219 | 0.447781264 |
| MNH18574.fas | Collinsella sp. AF18-8 | GCF_003459785.1 | 78.7363 | 0.427162707 |
| MNH18574.fas | Collinsella sp. AF18-8LB | GCF_003459795.1 | 78.8847 | 0.424025101 |
| MNH18574.fas | Collinsella sp. AF18-33LB | GCF_003459895.1 | 78.7766 | 0.429852084 |
| MNH18574.fas | Collinsella sp AF16-8[1] | GCF_003462695.1 | 78.2133 | 0.441057822 |
| MNH18574.fas | Collinsella sp AF1 5-5 1 | GCF 003462845.1 | 78.3798 | 0.436127297 |
| MNH18574.fas | Collinsella sp 0F02-10 | GCF_003463445.1 | 78.1552 | 0.449574182 |
| MNH18574.fas | Collinsella sp. AF 14-35 | GCF_003464565.1 | 78.233 | 0.441506051 |
| MNH18574.fas | Collinsella sp. AF11-11 | GCF_003465485.1 | 78.5023 | 0.448229494 |
| MNH18574.fas | Collinsella sp. AF04-24 | GCF_003465685.1 | 78.2561 | 0.447781264 |
| MNH18574.fas | Collinsella sp. TM 10-22 | GCF_003466125.1 | 78.5977 | 0.44285074 |
| MNH18574.fas | Collinsella sp. TM05-38 | GCF_003466445.1 | 78.673 | 0.444643658 |
| MNH18574.fas | Collinsella sp. AM44-11 | GCF_003467145.1 | 78.1328 | 0.454952936 |
| MNH18574.fas | Collinsella sp. AM38-1BH | GCF_003467545.1 | 78.278 | 0.436127297 |
| MNH18574.fas | Collinsella sp AM36-4AA | GCF_003467795.1 | 78.4335 | 0.443747199 |
| MNH18574.fas | Collinsella sp. AM40-7AC | GCF_003468125.1 | 78.2699 | 0.45091887 |
| MNH18574.fas | Collinsella intestinalis | GCF_003468845.1 | 80.8748 | 0.497086508 |
| MNH18574.fas | Collinsella sp. AM34-10 | GCF_003469185.1 | 78.3151 | 0.4513671 |
| MNH18574.fas | Collinsella sp. AM31-2AC | GCF_003469285.1 | 78.3023 | 0.449574182 |
| MNH18574.fas | Collinsella intestinalis | GCF_003469765.1 | 80.6798 | 0.46884805 |
| MNH18574.fas | Collinsella sp. AM28-11LB | GCF_003469895.1 | 78.5511 | 0.445988346 |
| MNH18574.fas | Collinsella sp. AM23-17 | GCF_003470945.1 | 78.2912 | 0.441506051 |
| MNH18574.fas | Collinsella sp AM20-15AC | GCF_003471405.1 | 78.2903 | 0.449125952 |
| MNH18574.fas | Collinsella sp AMI 5-2 | GCF_003471985.1 | 78.3407 | 0.439264904 |
| MNH18574.fas | Collinsella sp. AMI 0-48 | GCF_003472465.1 | 78.4948 | 0.446884805 |
| MNH18574.fas | Collinsella sp. AMI 0-32 | GCF_003472485.1 | 78.5379 | 0.445988346 |
| MNH18574.fas | Collinsella sp. AM10-27 | GCF_003472505.1 | 78.528 | 0.440161363 |
| MNH18574.fas | Collinsella sp. AMI 0-26 | GCF_003472525.1 | 78.5404 | 0.443298969 |

(continued)

|  |  |  | Ref | ANI | AF |
|---|---|---|---|---|---|
| MNH18574.fas | *Collinsella sp. AMI 3-34* | GCF_003473055.1 | 78.3622 | 0.452711788 |
| MNH18574.fas | *Collinsella sp. AM 10-11* | GCF_003473225.1 | 78.5674 | 0.443298969 |
| MNH18574.fas | *Collinsella sp. AM09-41* | GCF_003473245.1 | 78.4715 | 0.437920215 |
| MNH18574.fas | *Collinsella sp. AF38-3AC* | GCF_003474365.1 | 78.6451 | 0.471089198 |
| MNH18574.fas | *Collinsella sp. AF37-9* | GCF_003475115.1 | 78.4195 | 0.445540117 |
| MNH18574.fas | *Collinsella sp. AF31-11* | GCF_003475605.1 | 78.4633 | 0.451815329 |
| MNH18574.fas | Collinsella sp AF29-7AC | GCF_003475665.1 | 78.24 | 0.444643658 |
| MNH18574.fas | *Collinsella sp. AF08-23* | GCF_003479805.1 | 80.5627 | 0.50425818 |
| MNH18574.fas | Collinsella aerofaciens | GCF_003856815.1 | 78.6216 | 0.453608247 |
| MNH18574.fas | *Collinsella aerofaciens* | GCF_005576555.1 | 78.3102 | 0.448677723 |
| MNH18574.fas | *Collinsella aerofaciens* | GCF_005844325.1 | 78.3197 | 0.453608247 |
| MNH18574.fas | *Collinsella aerofaciens* | GCF_005845035.1 | 78.2259 | 0.449125952 |
| MNH18574.fas | *Collinsella bouchesdurho nensis* | GCF_005845055.1 | 77.535 | 0.382787987 |
| MNH18574.fas | *Collinsella sp. BA40* | GCF_008014645.1 | 85.4128 | 0.644554012 |
| MNH18574.fas | *Collinsella sp. WCA1-178- WT-3 (M2)* | GCF_009696305.1 | 78.29 | 0.428059166 |
| MNH18574.fas | *Collinsella sp. WCA1-178- WT-3 (MI)* | GCF_009696325.1 | 78.2792 | 0.429852084 |
| MNH18574.fas | *Collinsella ihuae* | GCF_900046475.1 | 78.8497 | 0.544150605 |
| MNH18574.fas | *Collinsella phocaeensis* | GCF_900119895.1 | 82.5374 | 0.543702376 |
| MNH18574.fas | *Collinsella bouchesdurho nensis* | GCF_900155365.1 | 77.6397 | 0.375616316 |
| MNH18574.fas | *Collinsella vaginalis* | GCF_900176655.1 | 78.007 | 0.472433886 |
| MNH18574.fas | *Collinsella provencensis* | GCF_900199705.1 | 78.7266 | 0.373375168 |
| MNH18574.fas | *Collinsella aerofaciens* | GCF_900461335.1 | 78.1629 | 0.455401165 |
| MNH18574.fas | *Collinsella aerofaciens* | GCF_901212465.1 | 78.3114 | 0.458538772 |
| MNH18574.fas | *Collinsella aerofaciens* | GCF_901212495.1 | 78.2628 | 0.445540117 |
| MNH18574.fas | *Collinsella aerofaciens* | GCF_902363035.1 | 78.5643 | 0.417301658 |
| MNH18574.fas | *Collinsella aerofaciens* | GCF_902363845.1 | 78.275 | 0.446436576 |
| MNH18574.fas | *Collinsella intestinalis* | GCF_902364775.1 | 80.6798 | 0.46884805 |
| MNH18574.fas | *Collinsella aerofaciens* | GCF_902364885.1 | 78.3149 | 0.448677723 |
| MNH18574.fas | *Collinsella aerofaciens* | GCF_902364945.1 | 78.2275 | 0.454504706 |
| MNH18574.fas | *Collinsella aerofaciens* | GCF_902374195.1 | 78.1866 | 0.448677723 |
| MNH18574.fas | *Collinsella tanakaei* | GCF_902374475.1 | 81.2267 | 0.556701031 |
| MNH18574.fas | *Collinsella ihuae* | GCF_902375985.1 | 78.8497 | 0.544150605 |
| MNH18574.fas | *Collinsella stercoris* | GCF_902377675.1 | 80.3409 | 0.518153294 |
| MNH18574.fas | *Collinsella aerofaciens* | GCF_902501405.1 | 78.0886 | 0.476916181 |
| MNH18574.fas | *Collinsella aerofaciens* | GCF_902501435.1 | 78.2797 | 0.461676378 |
| MNH18574.fas | *Collinsella intestinalis* | GCF_902501455.1 | 80.9018 | 0.467055132 |
| MNH18574.fas | *Collinsella aerofaciens* | GCF_902501475.1 | 78.2435 | 0.432989691 |
| MNH18574.fas | *Collinsella aerofaciens* | GCF_902501535.1 | 78.2892 | 0.455401165 |
| MNH18574.fas | *Collinsella aerofaciens* | GCF_902501595.1 | 78.0763 | 0.470640968 |

(continued)

|  |  | Ref | ANI | AF |
|---|---|---|---|---|
| MNH18574.fas | *Collinsella sp. AKJ07A* | GCF_902501965.1 | 78.9238 | 0.489018377 |

### 1.3.2 Phylogenetic tree of the strain MNH18574

First, the 16S rRNA gene sequences of all strains of the genus *Collinsella* to which MNH18574 belongs were retrieved from the LPSN database using the MEGA-X software. Then, the MUSCLE method was used to perform multiple sequence comparison between the above 16S sequences and those of the genus *Collinsella.* Finally, a phylogenetic tree was constructed using the maximum likelihood method. The results are shown in Fig. 8.

### 1.4 Genomic analysis of the strain MNH18574

### 1.4.1 Analysis of potential drug-resistance genes

Potential antibiotic resistance genes in the genome were analyzed using the RGI process (version: 4.2.2), in which the antibiotic resistance gene database was CARD (version: 3.0.0, https://card.mcmaster.ca/analyze/rgi). Detailed alignment information is shown in Table 4.

Table 4. List of drug-resistance gene information

| Strain gene | Resistance gene | Gene name | Alignment identity (%) |
|---|---|---|---|
| MNH18574_01644 | ARO:3000596 | ErmX | 95.89 |
| MNH18574 _01657 | ARO:3000194 | tetW | 93.26 |

### 1.4.2 Analysis of potential virulence genes

Potential virulence factors and related genes in the genome were analyzed by using NCBI blastp (version: 2.7.1+) to compare against the virulence factor database VFDB (http://www.mge.ac.cn/cgi-bin/VFs/v5/main.cgi, updated on September 19, 2019). Detailed comparison results are shown in Table 5.

Table 5. List of potential virulence genes of MNH18574

| Strain gene | VFDB gene | Gene name | Alignment identity (%) |
|---|---|---|---|
| MNH18574_00078 | VFG001376 | cps4L | 76.02 |
| MNH18574_00089 | VFG001374 | cps4J | 60.23 |

### 1.4.3 Analysis of potential primary metabolism gene clusters

Potential primary metabolism gene clusters in the genome were analyzed using gutSMASH5 (version: 1.0.0). Detailed comparison results are shown in Table 6.

Table 6. List of potential primary metabolism gene clusters of MNH18574

| Gene cluster range | Type | From | To | Most similar known gene cluster | Abbreviation | Similarity |
|---|---|---|---|---|---|---|
| Region 2.1 | TPP_AA_metabolism | 233241 | 260051 |  |  |  |
| Region 5.1 | Rnf_complexFlavoenzyme _sugar_catabolism | 1 | 30876 | Rnf complex C. sporogenes | RNF | 33% |
| Region 6.1 | Others_HGD_unassigned | 32771 | 57369 |  |  |  |
| Region 11.1 | Ech_complex | 21310 | 46606 | Ech complex C. sp. AF08-23 | ECH | 100% |

**1.5 Short-chain fatty acid (SCFA) assay of the strain MNH18574**

**1.5.1 Preparation of bacterial cells**

The strain MNH18574 was inoculated into TSB liquid medium, anaerobically cultured at 37 °C for 48 hours, centrifuged to collect the bacterial cells, which were stored at -86 °C for future use.

**1.5.2 Formulation of standards**

Acetic acid, propionic acid, butyric acid, isobutyric acid, valeric acid, isovaleric acid, and hexanoic acid standards were weighed, and formulated with ethyl acetate to form eight mixed standard concentration gradients: 0.1 μg/mL, 0.5 μg/mL, 1 μg/mL, 5 μg/mL, 10 μg/mL, 20 μg/mL, 50 μg/mL, and 100 μg/mL.
To 600 μL of the standard was added 25 μL of 4-methylvaleric acid at a final concentration of 500 μM as an internal standard, mixed well, and charged into an injection bottle for GC-MS detection. The injection volume was 1 μL, and the split ratio was 10:1. Split injection was used.

**1.5.3 Extraction of metabolites**

The sample was thawed on ice. 80 mg of the sample was pipetted into a 2 mL glass centrifuge tube, resuspended in 900 μL of 0.5% phosphoric acid, mixed well by shaking for 2 min, and centrifuged at 14,000 g for 10 min. 800 μL of the supernatant was pipetted, to which an equal amount of ethyl acetate was added for extraction, mixed well by shaking for 2 min, and centrifuged at 14,000 g for 10 min. 600 μL of the upper organic phase was pipetted, to which 4-methylvaleric acid at a final concentration of 500 μM was added as an internal standard, mixed well, and charged into an injection bottle for GC-MS detection. The injection volume was 1 μL, and the split ratio was 10:1. Split injection was used.

**1.5.4 Sample detection and analysis**

The samples were separated on an Agilent DB-WAX capillary column (30 m × 0.25 mm ID × 0.25 μm) gas chromatography system. Temperature programming: the initial temperature was 90 °C, raised to 120 °C at 10 °C/min, then to 150 °C at 5 °C/min, finally to 250 °C at 25 °C/min, and maintained for 2 min. The carrier gas was helium, with a flow rate of 1.0 mL/min.
Mass spectrometry analysis was performed on an Agilent 7890A/5975C gas chromatograph-mass spectrometer. The inlet temperature was 250 °C, the ion source temperature was 230 °C, the transmission line temperature was 250 °C, and the quadrupole temperature was 150 °C. An electron impact ionization (EI) source was used in full-scan and SIM scanning modes with an electron energy of 70 eV.
The chromatographic peak area and retention time were extracted using the MSD ChemStation software. A standard curve was plotted to calculate the content of short-chain fatty acids in the sample. The results are shown in Table 7.

Table 7. Results of short-chain fatty acid (SCFA) yields in the strain MNH18574

| SCFA Yield (μg/g) | Acetic acid | Butyric acid | Isovaleric acid | Valeric acid | Hexanoic acid | Propionic acid | Isobutyric acid |
|---|---|---|---|---|---|---|---|
| MNH18574 | 692.03 | 1.06 | 0.17 | 0.066 | 0.069 | 1.24 | 0.27 |

The results of short-chain fatty acid yields show that the strain of the present disclosure is highly productive of acetic acid.

**Example 2**

In this Example, the tumor inhibitory effect of the strain MNH18574 obtained in Example 1 was examined as follows:

**2.1 Regulation of macrophage immunoreactivity by the strain MNH18574**

A glycerol cryovial of the strain was thawed at 37 °C, and then inoculated onto an anaerobic blood plate under anaerobic conditions for activation. The activated strain was inoculated into MM01 liquid medium, anaerobically cultured, and then centrifuged. The bacterial pellet was resuspended in an appropriate amount of PBS, and subjected to MNH18574 flow cytometry in accordance with the instructions for use of the "Live/Dead Baclight Bacterial Viability Kits", to obtain strain samples that meet the experimental requirements.

THP-1 cells (Wuhan Pricella Biotechnology Co., Ltd.) were treated with PMA at a final concentration of 5 ng/mL for 48 hr such that they were differentiated into M0 macrophages.

M0 macrophages were co-incubated with the strain MNH18574 for 24 hr. The strain MNH18574 was added according to the ratio of MOI (viable bacterial count: cell count) = 10:1. Also, a control group of M1 macrophages (derived from M0 macrophages by induction with 20 ng/ml IFN$\gamma$ + 10 pg/ml LPS for 24 hr) was set up. After anaerobic incubation for 1 hr, a mixture of antibiotics (1 mg/mL Ampicillin; 5 mg/mL Streptomycin; and 1 mg/mL Colistin) was added to kill the bacteria. After culturing for an additional 23 h, the supernatant was collected.

The concentrations of the cytokines MCP-1, IL-1$\beta$, MIG, IL-6, and TNF$\alpha$ in the supernatant were detected by flow cytometry after the supernatant sample was allowed to interact with the assay proteins using the BD™ Cytometric Bead Array (CBA) Human Soluble Protein Master Buffer Kit according to the manufacturer's instructions.

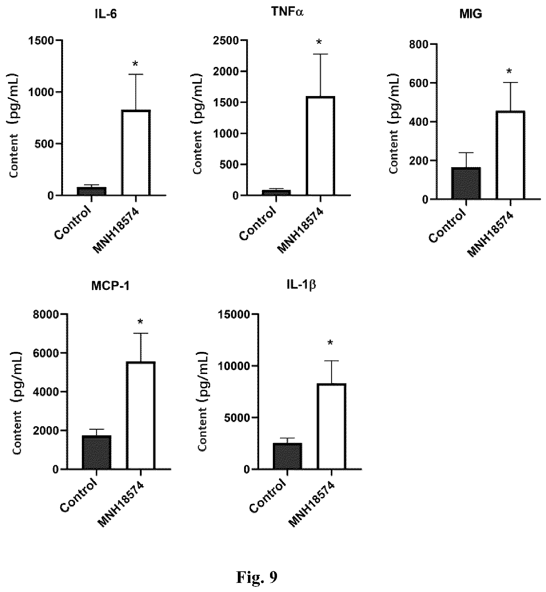

The results showed that after co-culture of macrophages with MNH18574, the macrophages clearly exhibited characteristics of differentiation towards M1-type macrophages. In particular, the contents of the chemokines MIG and MCP-1 were significantly upregulated, and the contents of the pro-inflammatory factors IL-1$\beta$, IL-6, and TNF$\alpha$ were significantly upregulated, as shown in Fig. 9.

### 2.2 Regulation of primary PBMC immunoreactivity by the strain MNH18574

Previous studies have shown that the components of gut microbes have a profound influence on the surrounding immune system, and that gut microbes have a significant impact on the effect of tumor immunotherapy in cancer patients, and are key components of anti-tumor immunity and therapeutic efficacy. Gut microbes and immune cells are capable of interacting and co-regulating the human immune system. Gut strains can induce primary PBMCs to differentiate into different cell types and secrete different inflammatory factors, and therefore can be used for screening of strains with immunoregulatory effect.

(ii) Experimental methods:

Test strain: A glycerol cryovial of the strain MNH18574 was thawed at 37 °C, and then inoculated onto an anaerobic blood plate in an anaerobic workstation for activation. The activated strain was inoculated into MM01 liquid medium, anaerobically cultured, and then centrifuged. The bacterial pellet was resuspended in an appropriate amount of PBS, and subjected to MNH18574 flow cytometry in accordance with the instructions for use of the "Live/Dead Baclight Bacterial Viability Kits", to obtain strain samples that meet the experimental requirements.

The commercial primary PBMCs (purchased from TPCS, Batch No. A19Z289100) were recovered and cultured in PRMI1640 complete medium (containing 10% heat-inactivated FBS, 1% L-glutamine, 0.1% ps (a penicillin-streptomycin mixture), and 10 mg/ml DNase for avoiding agglutination).

The strain MNH18574 was co-incubated with Primary PBMCs for 24 h, and then the supernatant was collected. The concentrations of the cytokines IFN$\gamma$, TNF$\alpha$, IL-1$\beta$, IP-10 (CXCL-10), RANTES (CCL5), MIG (CXCL-9), IL-6, IL-10, and MCP-1 (CCL2) in the supernatant were detected by flow cytometry after the supernatant sample was allowed to interact with the assay proteins using the BD™ Cytometric Bead Array (CBA) Human Soluble Protein Master Buffer Kit according to the manufacturer's instructions.

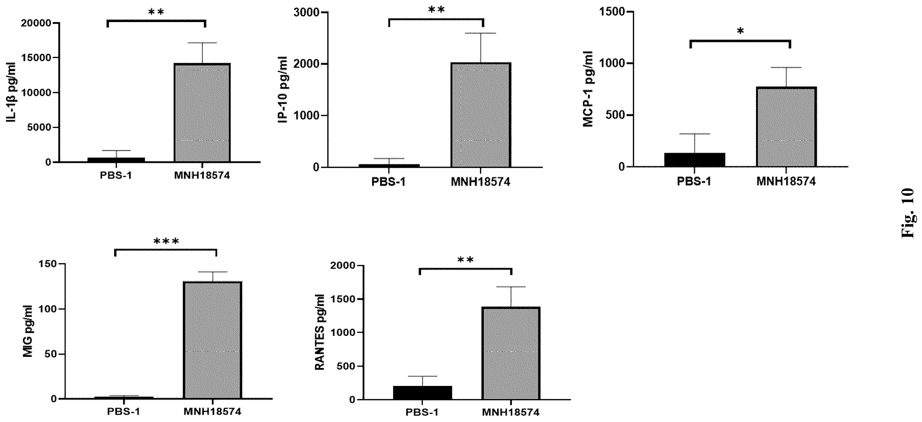

The results showed that MNH18574 significantly induced an increase in the inflammatory factor IL-1$\beta$ and the chemokines IP-10 (CXCL-10), MCP-1 (CCL2), MIG (CXCL-9), and RANTES (CCL5); induced a tendency of enhanced expression of the inflammatory factors TNF$\alpha$, IL-6, and IFN$\gamma$; and reduced the expression of the anti-inflammatory factor IL-10, as shown in Fig. 10.

### 2.3 Experiment for verifying the therapeutic effect of the strain MNH18574 on lung cancer

In order to verify whether the strain MNH18574 has a therapeutic effect on a tumor, a lung cancer growth inhibition experiment was carried out using a mouse syngeneic tumor model. This experimental protocol has been ethically reviewed by the Laboratory Animal Care and Use Committee of Moon Biotech.

Test strain: A glycerol cryovial of the strain MNH18574 was thawed at 37 °C, and then inoculated onto an anaerobic blood plate in an anaerobic workstation for activation. The activated strain was inoculated into MM01 liquid medium and anaerobically cultured to obtain a sufficient number of cultures. The cultured bacterial solution was centrifuged, concentrated, and then resuspended in a solvent to afford a test article with a purity and viable bacterial count ($1 \times 10^9$ - $2 \times 10^{10}$ CFU/mL) meeting the requirements of animal experiments.

Tumor cells: LLC1 mouse lung cancer cells, from Wuhan Pricella Biotechnology Co., Ltd., Article No. CL-0140.

Experimental animal: C57BL/6J mice, aged 5 weeks, 40 mice in total, purchased from Guangdong GemPharmatech Co., Ltd.

Animal experiment: The mice were normally fed. Upon completion of the quarantine period, the mice were subcutaneously inoculated with LLC1 lung cancer cells in an amount of cell inoculation of $2 \times 10^6$/mL, 0.1-0.2 mL/animal, to create an ectopic syngeneic tumor model. When the average tumor volume reached 80-100 mm$^3$, the mice was randomized into 6 groups according to tumor volume stratification. Gavage administration was initiated on the day of grouping (D1) for Group A (Negative Control) and Group B (MNH18574). The control group was given a solvent, and Group B was given the MNH18574 bacteria. The volume of the gavage was 0.2 mL/animal/dose, and the frequency of administration was once per day. General observation was carried out once a day during the quarantine period and after the daily dose during the drug administration. The animals were weighed upon arrival and at the end of the quarantine period. After tumor inoculation, the animals were weighed twice a week, and the tumor diameter was measured every 2 days. When the average tumor volume was $\geq$ 1000 mm$^3$, the tumor diameter was measured once a day, and the tumor growth was recorded.

The endpoint of this experiment was as follows: the average tumor volume in any group was $\geq$ 1000 mm$^3$ and the tumor volume in Group B was significantly smaller than that in Group A, or the average tumor growth inhibition rate (TGI) in this group was $\geq$ 20% as compared to Group A; or the average tumor volume in any group of mice was greater than 2000 mm$^3$. General clinical observation, body weight monitoring, and tumor volume (mm$^3$) measurement were conducted during the experimental process. After the experimental endpoint was reached, all the surviving mice were dissected and sampled, tumor weight was measured and tumor volume was calculated, and finally, statistical analyses and comparisons were performed for tumor volume curve change (Tumor volume), tumor volume at the endpoint, and tumor growth inhibition rate (TGI) at the endpoint. Tumor growth inhibition rate = (Average volume in the control group - Volume in the experimental group) / Average volume in the control group $\times$ 100%. All data were expressed as mean $\pm$ SD, and plotted and statistically analyzed using the GraphPad Prism 8.0.2 software. For pairwise comparisons, t-test (Student's t test) was used. Two-way analyses were performed by 2-way ANOVA with Sidak multiple comparison. The significance of difference was indicated by *: * $p < 0.05$, ** $p < 0.01$, *** $p < 0.001$, **** $p < 0.0001$.

At the experimental endpoint (D21), the surviving mice were dissected. The results are summarized in Table 8.

Table 8. Overall statistics of mice at the experimental endpoint

| | Number of mice with successful tumor inoculation | Number of mice at experimental endpoint | Number of euthanized mice | Average body weight prior to dissection (g) | Average size of dissected tumors (mm$^3$) | TGI at experimental endpoint (%) |
|---|---|---|---|---|---|---|
| Group A (Negative Control) | 12 | 11 | 1 | 23.34 | 1136.39 | 0% |
| Group B (MNH18574) | 12 | 11 | 1 | 23.72 | 831.78 | 28.7% |

The results showed that the statistical analysis results of the tumor volume curve of Group B (MNH18574) were smaller than those of Group A (Negative Control) during the experiment. At the experimental endpoint, the average tumor volume of control mice was 1136.39 mm$^3$; while the average tumor volume of MNH18574-treated mice was 831.78 mm$^3$. The tumor volume of MNH18574-treated mice was significantly smaller than that of the control group (as shown in Table 8). At the experimental endpoint, the tumor growth inhibition rate (TGI) of Group B (MNH18574) was 28.7%.

**2.4 Experiment for verifying the therapeutic effect of the strain MNH18574 in combination with anti-PD-1 antibody on lung cancer**

In order to verify the therapeutic effect of the strain MNH18574 in combination with an anti-PD-1 antibody (BioXcell) on a tumor, a lung cancer growth inhibition experiment was carried out using a mouse syngeneic tumor model. This experimental protocol has been ethically reviewed by the Laboratory Animal Care and Use Committee of Moon Biotech.

Test strain: A glycerol cryovial of the strain MNH18574 was thawed at 37 °C, and then inoculated onto an anaerobic blood plate in an anaerobic workstation for activation. The activated strain was inoculated into MM01 liquid medium and anaerobically cultured to obtain a sufficient number of viable bacteria. The cultured bacterial solution was centrifuged, concentrated, and then resuspended in a solvent to afford a test article with a purity and viable bacterial count

$(1.30 \times 10^{10} - 1.5 \times 10^{10}$ CFU/mL) meeting the requirements of animal experiments.

Tumor cells: LLC1 mouse lung cancer cells, from Wuhan Pricella Biotechnology Co., Ltd., Article No. CL-0140.

Experimental animal: C57BL/6J mice, aged 5-6 weeks, 40 mice in total, purchased from Guangdong Gem-Pharmatech Co., Ltd.

Animal experiment: The mice were normally fed. Upon completion of the quarantine period, the mice were subcutaneously inoculated with LLC1 lung cancer cells in an amount of cell inoculation of $2 \times 10^6$/mL, 0.1 mL/animal, to create an ectopic syngeneic tumor model. When the average tumor volume reached 60-100 mm$^3$, the mice was randomized into 10 groups according to tumor volume stratification: Group A (Negative Control), Group B (Positive Control, anti-PD-1), Group E (MNH18574 & anti-PD-1), and Groups C-D and F-J using other test drugs. The dosing was initiated on the day of grouping. Group A was given a solvent and physiological saline, Group B was given a solvent and an anti-PD-1 antibody, and Group E was given MNH18574 and the anti-PD-1 antibody. The solvent and MNH18574 were administered by gavage, with a volume of 0.15-0.25 mL/animal, and a frequency of once a day. The anti-PD-1 antibody and physiological saline were administered by intraperitoneal injection, at a dose of 9.5-10.5 mg/kg, and a frequency of once every 3 days, for a total of 5 times. The animals were weighed upon arrival and at the end of the quarantine period. After tumor inoculation, the animals were weighed twice a week, including the day of intraperitoneal injection. The day of tumor cell inoculation was D1. The tumor diameter was measured once a day from D5 to the day before grouping, once every 3 days after grouping, and once a day upon the average tumor volume $\geq$ 1000 mm$^3$, and the tumor growth was recorded.

The experiment might be terminated when the average tumor volume in any group was $\geq$ 1000 mm$^3$, and the tumor volume in any one of Groups C to J was significantly smaller than that in Group A and significantly smaller than that in Group B, or the average tumor growth inhibition rate (TGI) in this group was $\geq$ 20% as compared to Group B; or the average tumor volume in any group was greater than 2000 mm$^3$. General clinical observation, body weight monitoring, and tumor volume (mm$^3$) measurement were conducted during the experimental process. After the experimental endpoint was reached, all the surviving mice were dissected and sampled, tumor weight was measured and tumor volume was calculated, and finally, statistical analyses and comparisons were performed for tumor volume curve change (Tumor volume), tumor volume at the endpoint, tumor weight at the endpoint, and tumor growth inhibition rate (TGI) at the endpoint. Tumor growth inhibition rate = (Average volume in the control group - Volume in the experimental group) / Average volume in the control group $\times$ 100%. All data were expressed as mean $\pm$ SD, and plotted and statistically analyzed using the GraphPad Prism 8.0.2 software. For pairwise comparisons, t-test (Student's t test) was used. Two-way analyses were performed by 2-way ANOVA with Sidak multiple comparison. The significance of difference was indicated by *: * $p < 0.05$, ** $p < 0.01$, *** $p < 0.001$, **** $p < 0.0001$.

At the experimental endpoint (D20), the surviving mice were dissected and the tumor tissues were weighed. The results are summarized in Table 9.

Table 9. Overall statistics of mice at the experimental endpoint

| Group | Number of mice enrolled | Number of mice at experimental endpoint | Number of euthanized mice | Average weight of dissected Tumors (g) | Average body weight prior to dissection (g) | Average tumor size at endpoint (mm$^3$) |
|---|---|---|---|---|---|---|
| Group A (Negative Control) | 8 | 8 | 0 | 1.02 | 23.86 | 1007.22 |
| Group B (Positive Control, anti-PD-1 antibody) | 8 | 8 | 0 | 0.84 | 24.43 | 715.00 |
| Group E (MNH18574 & anti-PD-1 antibody) | 8 | 8 | 0 | 0.39 | 23.06 | 384.07 |

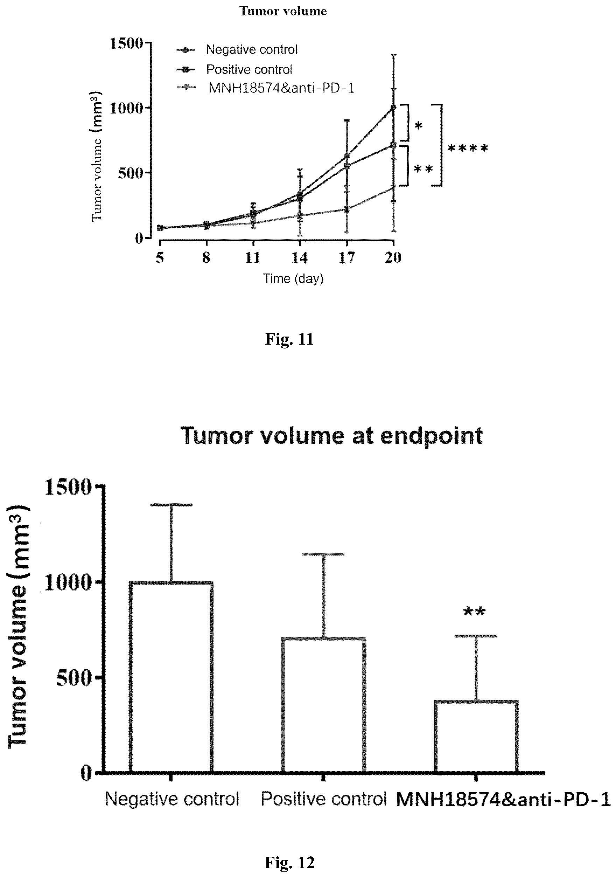

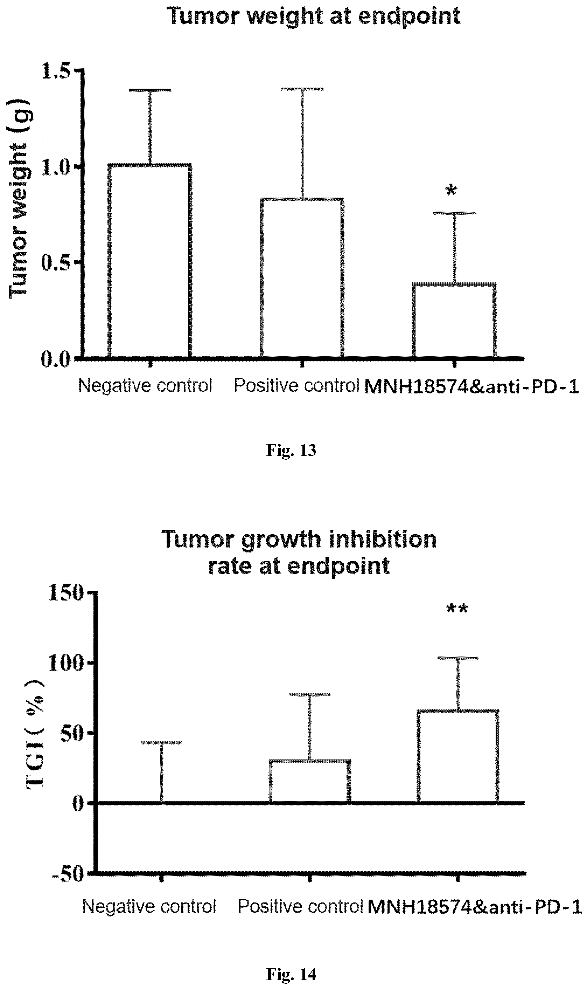

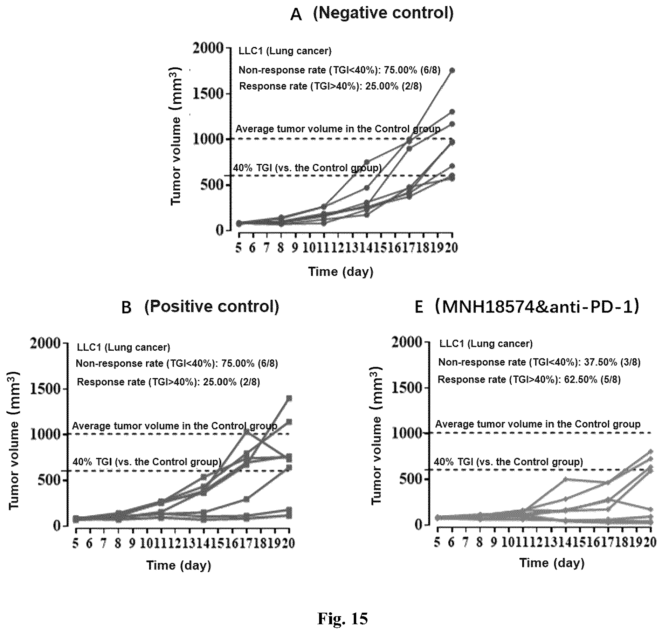

The results showed that the statistical analysis results of the tumor volume curve of Group E (MNH18574 & anti-PD-1) were significantly smaller than those of Group A (Negative Control) and Group B (Positive Control) during the experiment (as shown in Fig. 11). At the experimental endpoint, the average tumor volume of mice in the Negative Control group was 1007.22 mm$^3$, and the average weight of tumor tissues was about 1.02 g. The average tumor volume of mice in the Positive Control group was 715.00 mm$^3$, and the average weight of tumor tissues was about 0.84 g. The average tumor volume of mice in the MNH18574 & anti-PD-1 antibody-treated group was 384.07 mm$^3$, and the average weight of tumor tissues was about 0.39 g. The tumor volume and tumor weight of mice in the MNH18574 & anti-PD-1 antibody-treated group were significantly smaller than those in the control group (Figs. 12-13). At the experimental endpoint, the tumor growth inhibition rate (TGI) was 31.4% for Group B (Positive Control) and 67.1% for Group E (MNH18574 & anti-PD-1), which was higher than that of the Positive Control group (Fig. 14). At the experimental endpoint, the response rate was 25.0% for Group A (Negative Control), 25.0% for Group B (Positive Control), and 62.5% for Group E (MNH18574 & anti-PD-1), suggesting that the combination of MNH18574 with an anti-PD-1 antibody increased the response rate of tumors to the treatment (Fig. 15). The experimental results showed **no significant difference in the therapeutic effect of the anti-PD-1 antibody on lung cancer relative to the negative control group** in terms of the tumor weight of dissected mice, the tumor volume at the endpoint, or the TGI at the endpoint, **meaning that lung cancer is a clinically refractory tumor.**

The anti-PD-1 antibody is an anti-tumor inhibitor commonly used in clinical practice. Comparison of A, B and E in Fig. 15 shows that after 14 days of treatment with the anti-PD-1 antibody in Group B, the high expression of PD-L1 will lead to continuous activation of the PD-1/PD-L1 signaling pathway, which causes tumor cells to evade the surveillance and killing of the immune system, thereby promoting tumor growth and exacerbating the drug resistance of tumors.

However, the *Collinsella* sp. of the present application in combination with an anti-PD-1 antibody can enhance the tumor-killing effect of the body's immune system, and can improve the anti-tumor effect of an immunosuppressive agent. Furthermore, it can be seen from E of Fig. 15 that, on days 15-18 of treatment with the strain in combination with the anti-PD-1 antibody, as compared to the group treated with the anti-PD-1 antibody alone, the strain of the present application can cause reduced tumor volume, indicating that the strain of the present application can effectively reduce the resistance of the organism to the anti-PD-1 antibody, and can effectively achieve anti-tumor effects.

Since a new species of *Collinsella* was screened out, the present disclosure can improve the anti-tumor effect of an immunosuppressive agent by enhancing the tumor-killing effect of the body's immune system by using the strain in combination with an anti-PD-1 antibody. Furthermore, the strain of the present disclosure can effectively reduce the resistance of the organism to the anti-PD-1 antibody, and can effectively achieve anti-tumor effects.

### 2.5 Experiment for verifying the therapeutic effect of the strain MNH18574 in combination with anti-PD-1 antibody on pancreatic cancer

In order to verify the therapeutic effect of the strain MNH18574 in combination with an anti-PD-1 antibody (BioXcell) on a tumor, a lung cancer growth inhibition experiment was carried out using a mouse syngeneic tumor model. This experimental protocol has been ethically reviewed by the Laboratory Animal Care and Use Committee of Moon Biotech.

Test strain: A glycerol cryovial of the strain MNH18574 was thawed at 37 °C, and then inoculated onto an anaerobic blood plate in an anaerobic workstation for activation. The activated strain was inoculated into MM01 liquid medium and anaerobically cultured to obtain a sufficient number of viable bacteria. The cultured bacterial solution was centrifuged, concentrated, and then resuspended in a solvent to afford a test article with a purity and viable bacterial count $(1.30 \times 10^{10} - 1.5 \times 10^{10}$ CFU/mL) meeting the requirements of animal experiments.

Tumor cells: KPC mouse pancreatic cancer cells, from Future (Suzhou) Biotechnology Co., Ltd., Article No. FNC0071.

Experimental animal: female C57BL/6J mice, aged 7-8 weeks, purchased from Guangdong GemPharmatech Co., Ltd.

Animal experiment: The mice were normally fed. Upon completion of the quarantine period, the mice were subcutaneously inoculated with KPC pancreatic cancer cells in an amount of cell inoculation of $3 \times 10^6$/mL, 0.1 mL/animal, to create an ectopic syngeneic tumor model. When the average tumor volume reached 60-100 mm$^3$, the mice was randomized into 10 groups according to tumor volume stratification: Group A (Negative Control), Group B (Positive Control, anti-PD-1), Group E (MNH18574 & anti-PD-1), and Groups C-D and F-J using other test drugs. The dosing was initiated on the day of grouping. The negative control PBS-Cys (Gly) and MNH18574 were administered by gavage. When the average tumor volume in the negative control Group A reached to 120-150 mm$^3$ or after 7 days of gavage administration, the negative control, physiological saline, and the positive control, an anti-PD-1 antibody, were administered by intraperitoneal injection. Group A was given a solvent and physiological saline, Group B was given a solvent and the anti-PD-1 antibody, and Group E was given MNH18574 and the anti-PD-1 antibody. The solvent and MNH18574 were administered by gavage, with a volume of 0.15-0.25 mL/animal/dose, and a frequency of once a day. The anti-PD-1 antibody and physiological saline were administered by intraperitoneal injection, at a dose of 9.5-10.5 mg/kg, and a frequency of once every 3 days, for a total of 6 times. The animals were weighed upon arrival and at the end of the quarantine period. After tumor inoculation, the animals were weighed twice a week, including the day of intraperitoneal injection. The day of tumor cell inoculation was D1. The tumor diameter was measured once a day from D5 to the day before grouping, once every 3 days after grouping, and once every 2 days upon the average tumor volume $\geq 600$ mm$^3$, and the tumor growth was recorded.

The experiment might be terminated when the average tumor volume in any group was $\geq 600$ mm$^3$, and the tumor volume in any one of Groups C to J was significantly smaller than that in Group A and significantly smaller than that in Group B, or the average tumor growth inhibition rate (TGI) in this group was $\geq 20\%$ as compared to Group B; or the average tumor volume in any group was greater than 2000 mm³. General clinical observation, body weight monitoring, and tumor volume (mm³) measurement were conducted during the experimental process. After the experimental endpoint was reached, all the surviving mice were dissected and sampled, tumor weight was measured and tumor volume was calculated, and finally, statistical analyses and comparisons were performed for tumor volume curve change (Tumor volume), tumor volume at the endpoint, tumor weight at the endpoint, and tumor growth inhibition rate (TGI) at the endpoint. Tumor growth inhibition rate = (Average volume in the control group - Volume in the experimental group) / Average volume in the control group × 100%. All data were expressed as mean ± SD, and plotted and statistically analyzed using the GraphPad Prism 8.0.2 software. For pairwise comparisons, t-test (Student's t test) was used. Two-way analyses were performed by 2-way ANOVA with Sidak multiple comparison. The significance of difference was indicated by *: * $p < 0.05$, ** $p < 0.01$, *** $p < 0.001$, **** $p < 0.0001$.

At the experimental endpoint (D33), the surviving mice were dissected and the tumor tissues were weighed. The results are summarized in Table 10:

Table 10. Overall statistics of mice at the experimental endpoint

| Group | Number of mice enrolled | Number of mice at experimental endpoint | Number of euthanized mice | Average weight of dissected Tumor (g) | Average body weight prior to dissection (g) | Average tumor size at endpoint (mm³) |
|---|---|---|---|---|---|---|
| Group A (Negative Control) | 8 | 8 | 0 | 1.05 | 21.26 | 958.42 |
| Group B (Positive Control, anti-PD-1) | 8 | 8 | 0 | 1.03 | 22.52 | 871.39 |
| Group E (MNH18574 & anti-PD-1 antibody) | 8 | 8 | 0 | 0.73 | 20.98 | 682.49 |

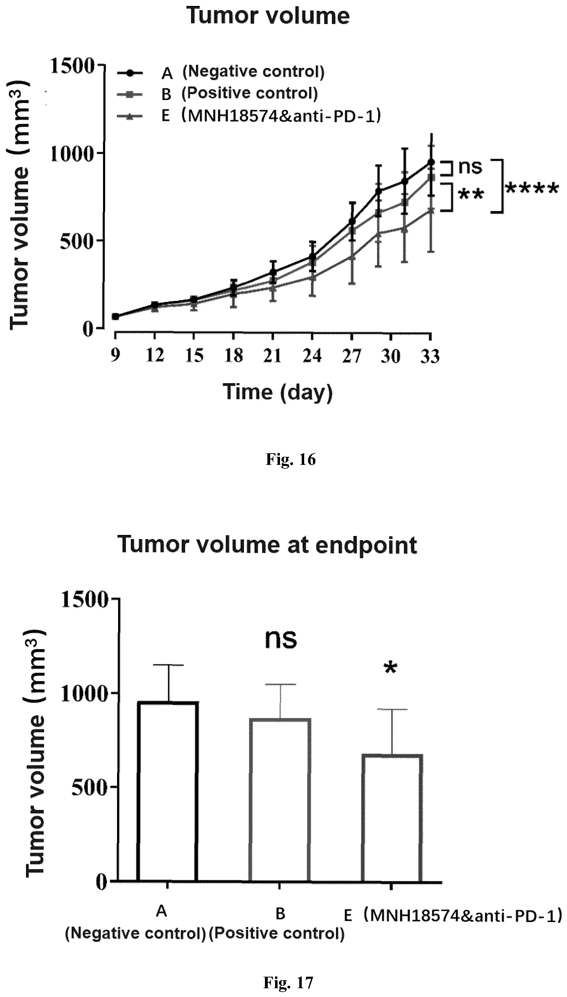

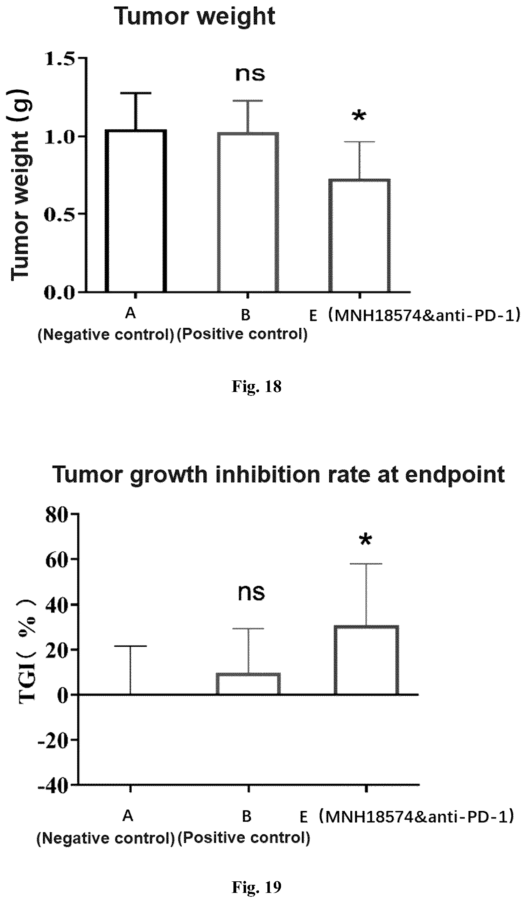

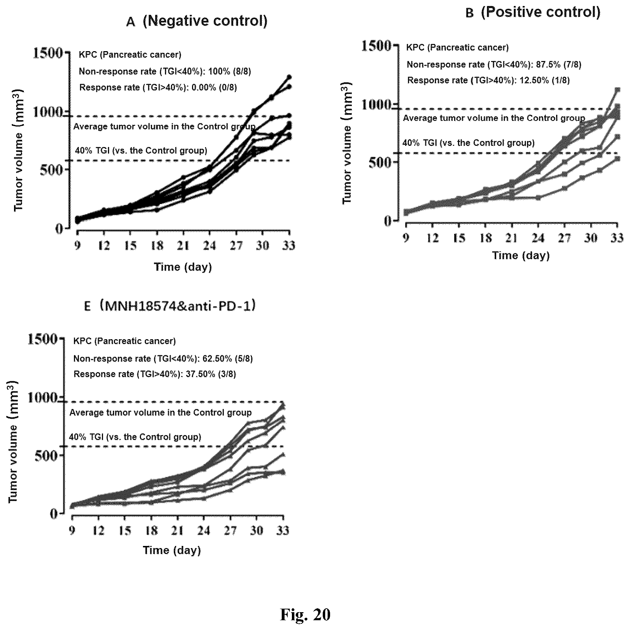

The results showed that the statistical analysis results of the tumor volume curve of Group E (MNH18574 & anti-PD-1) were significantly smaller than those of Group A (Negative Control) and Group B (anti-PD-1) during the experiment (as shown in Fig. 16). At the experimental endpoint, the average tumor volume of mice in the Negative Control group was 958.42 mm³, and the average weight of tumor tissues was about 1.05 g. The average tumor volume of mice in the Positive Control group was 871.39 mm³, and the average weight of tumor tissues was about 1.03 g. The average tumor volume of mice in the MNH18574 & anti-PD-1 antibody-treated group was 682.49 mm³, and the average weight of tumor tissues was about 0.73 g. The tumor volume and tumor weight of mice in the MNH18574 & anti-PD-1 antibody-treated group were significantly smaller than those in the control group (Figs. 17-18). At the experimental endpoint, the tumor growth inhibition rate (TGI) was 9.78% for Group B (anti-PD-1) and 30.99% for Group E (MNH18574 + anti-PD-1), which was higher than that of the positive control group (Fig. 19). At the experimental endpoint, the response rate was 0.00% for Group A (Negative Control), 12.5% for Group B (anti-PD-1), and 37.5% for Group E (MNH18574 + anti-PD-1). The combination of MNH18574 with the anti-PD-1 antibody increased the response rate of tumors to the treatment (Fig. 20).

Fig. 16 shows a graph of tumor volumes in mice provided in this Example. As shown in Fig. 16, the statistical analysis results of the tumor volume curve of Group E (MNH18574 + anti-PD-1) were significantly smaller than those of Group A (Negative Control) and Group B (anti-PD-1) during the experiment. The data in the graph are shown as mean ± standard deviation (Mean ± SD). The statistical analysis was performed using 2-way ANOVA with Sidak multiple comparison. The significance of difference was indicated by *: * p < 0.05.

Fig. 17 shows a graph comparing the tumor volumes in mice at the experimental endpoint provided in this Example. As shown in Fig. 17, the statistical analysis results of the tumor volumes in mice at the endpoint in Group E (MNH18574 + anti-PD-1) were smaller than those in Group A. The data in the graph are shown as mean ± standard deviation (Mean ± SD). The statistical analysis was performed using one-way ANOVA with Dunnet's multiple comparison. The significance of difference was indicated by *: * p < 0.05, ** p < 0.01, not significant, not indicated.

Fig. 18 shows a graph comparing the tumor weights in mice at the experimental endpoint provided in this Example. As shown in Fig. 18, the statistical analysis results of the tumor volumes in mice at the endpoint in Group E (MNH18574 + anti-PD-1) were smaller than those in Group A. The data in the graph are shown sa mean ± standard deviation (Mean ± SD). The statistical analysis was performed using one-way ANOVA with Dunnet's multiple comparison. The significance of difference was denoted by *: * p < 0.05, ** p < 0.01, not significant, not indicated.

Fig. 19 shows a graph comparing the tumor growth inhibition rates of mice at the experimental endpoint in the experiment provided in this Example. As shown in Fig. 19, the statistical analysis results of the tumor volumes of mice at the endpoint in Group E (MNH18574 + anti-PD-1) were greater than those of Group A. The data in the graph are shown as mean $\pm$ standard deviation (Mean $\pm$ SD). The statistical analysis was performed using one-way ANOVA with Dunnet's multiple comparison. The significance of difference was indicated by *: * $p < 0.05$, ** $p < 0.01$.

As shown in Fig. 19, at the experimental endpoint, the tumor growth inhibition rate (TGI) was 9.78% for Group B (anti-PD-1) and 30.99% for Group E (MNH18574 + anti-PD-1), which was significantly higher than that of the positive control group.

Fig. 20 shows a graph of response rates of mice at the experimental endpoint provided in this Example. As shown in Fig. 20, at the experimental endpoint, the response rate was 0.0% for Group A (Negative Control), 12.5% for Group B (Positive Control), and 37.5% for Group D (MNH18574 + anti-PD-1). The data in the graph are shown as the tumor volume ($mm^3$) in a single mouse.

The experimental results showed no significant difference in the therapeutic effect of the anti-PD-1 antibody on pancreatic cancer relative to the negative control group in terms of the tumor weight of dissected mice, the tumor volume at the endpoint, or the TGI at the endpoint, meaning that pancreatic cancer is a clinically refractory tumor. The anti-PD-1 antibody is an anti-tumor inhibitor commonly used in clinical practice. After 24 days of continuous treatment with the anti-PD-1 antibody, the high expression of PD-L1 will lead to continuous activation of the PD-1/PD-L1 signaling pathway, which causes tumor cells to evade the surveillance and killing of the immune system, thereby promoting tumor growth and exacerbating the drug resistance of tumors.

However, the *Collinsella* sp. of the present application in combination with an anti-PD-1 antibody can enhance the tumor-killing effect of the body's immune system, and can improve the anti-tumor effect of an immunosuppressive agent. On days 29-32 of treatment with the strain in combination with the anti-PD-1 antibody, as compared to the group treated with the anti-PD-1 antibody alone, the strain of the present application can cause reduced tumor volume, indicating that the strain of the present application can effectively reduce the resistance of the organism to the anti-PD-1 antibody, and can effectively achieve anti-tumor effects.

Since a new species of *Collinsella* was screened out, the present disclosure can improve the anti-tumor effect of an immunosuppressive agent by enhancing the tumor-killing effect of the body's immune system by using the strain in combination with an anti-PD-1 antibody. Furthermore, the strain of the present disclosure can effectively reduce the resistance of the organism to the anti-PD-1 antibody, and can effectively achieve anti-tumor effects.

**Example 3: Immunoassay of the strain MNH18574 in combination with anti-PD-1 antibody in a lung cancer treatment model**

**Principle of the experiment:**

After a tissue is prepared into a single cell suspension and labeled with a specific molecule carrying a fluorescent molecule marker, the proportion of cells expressing the specific molecule can be detected by flow cytometry. The proportional distribution of immune cells reflects the immune status of mice to a certain extent.

**Experimental methods:**

1. Preparation of tumor single cell suspension and cell counting

The lung cancer tumor tissue from the experiment for verifying the therapeutic effect of MNH18574 in combination with an anti-PD-1 antibody on lung cancer in Example 2 was subjected to surface rinsing with PBS, cut into small pieces, and pulverized. The pulverized tumor tissue was placed into a 5 mL Collagenase IV digestion solution system (1 mg/mL Collagenase IV, 0.1 mg/mL DNase, and 10% FBS) and digested at 37 °C for 30 min. The digested tissue solution was filtered through a 70 $\mu$M filter membrane to obtain a mouse tumor single cell suspension. The total number of cells in the tissue was counted by on-machine detection.

2. Staining for T-cell cytokines on the surface of tumor cells

The mouse tumor single cell suspension was stimulated with a cytokine stimulating solution (containing PMA, Ionomycin, and BFA) for 4 h. The dyes (corresponding antibody combinations) Live/Fc and T-Surface were respectively added for cell surface staining. Then, the suspension was treated with a fixative and a membrane-rupturing solution according to the instructions of the detection kit, followed by staining for the cytokines IFN-$\gamma$ and TNF-$\alpha$. The stained cells were resuspended in PBS and subjected to on-machine detection.

3. Statistical analysis

The flow cytometry data were processed by CyExpert and statistically processed by Graphpad PrisM V9. Statistical analysis was performed by one-way ANOVA with Dunnet's multiple comparison: ns, not significant, * p < 0.05, ** p < 0.01, *** p < 0.001. Correlation analysis was performed by linear regression, in which Pearson r reflected the correlation, and P value was used for statistical test.

**Experimental results:**

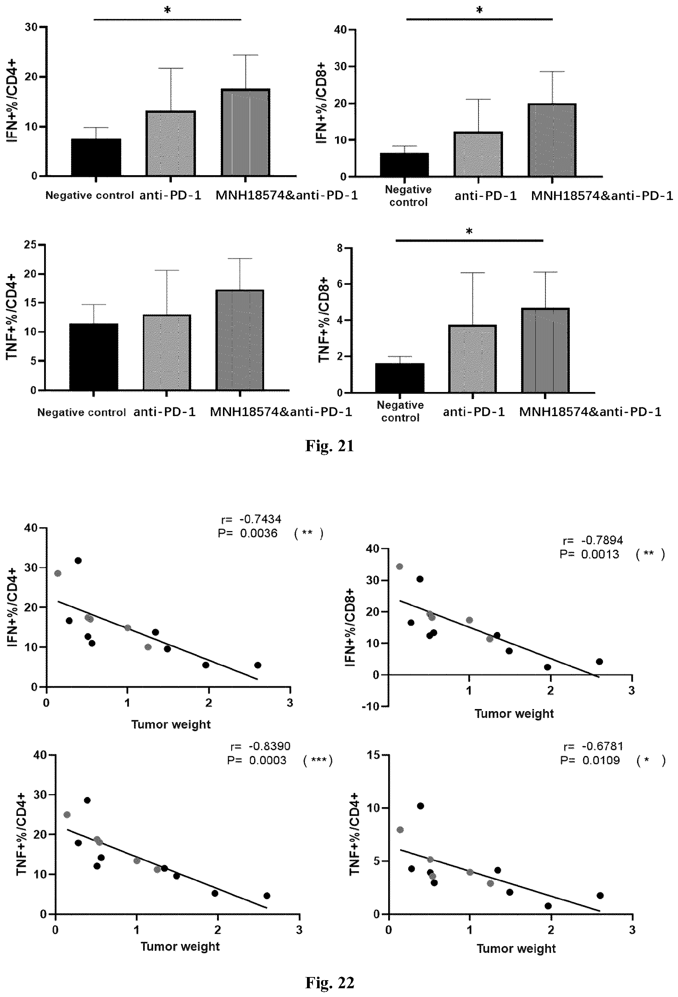

The experimental results are shown in Fig. 21. As compared to the Control group, MNH18574 in combination with an anti-PD-1 antibody significantly promoted a significant increase in the proportions of IFN-γ+CD4+, IFN-γ+CD8+, and TNF-α+CD8+ T cells in tumor tissues. That is, the strain combined with the anti-PD-1 antibody can significantly upregulate the proportions of IFN-$\gamma^+$CD4$^+$, IFN-$\gamma^+$CD8$^+$, and TNF-$\alpha^+$CD8$^+$ T cells in tumor tissues. In addition, the relationship between these cell proportions and tumor weights was further evaluated by a pharmacodynamic correlation analysis. The experiment for verifying the therapeutic effect of MNH18574 in combination with an anti-PD-1 antibody on lung cancer in Example 2 showed that the tumor weight in the mice treated with MNH18574 in combination with the anti-PD-1 antibody was decreased as compared to the mice treated with the anti-PD-1 antibody alone, and significantly decreased relative to the control group, as shown in Fig. 22. In the comparison of MNH18574 + anti-PD-1 vs. anti-PD-1, the proportions of CD4+IFN-γ+, CD8+IFN-γ+, CD4+TNF-α+, and CD8+TNF-α+ cells were significantly negatively correlated with the tumor weight, and the strain combined with the anti-PD-1 antibody resulted in a significant increase in the proportions of CD4+IFN-γ+, CD8+IFN-γ+, CD4+TNF-α+, and CD8+TNF-α+ cells, which were consistent with the tumor pharmacodynamic results. These results indicate that MNH18574 has a role in promoting T cell activation and increasing the anti-tumor effect of an anti-PD-1 antibody.

**Example 4: Effect of the strain MNH18574 in ameliorating metabolic diseases**

In this Example, the effect of the strain MNH18574 obtained in Example 1 in ameliorating metabolic diseases was investigated as follows:

*4.1 In vivo* experiment of the strain MNH18574 on liver and kidney functions and related diseases in a high-fat diet-induced obese mouse model

An experiment on the amelioration of liver and kidney functions and related diseases by MNH18574 was conducted using a high-fat diet-induced obese mouse model. This experiment has been ethically reviewed by the Laboratory Animal Care and Use Committee of Moon Biotech.

(1) Experimental animal: C57BL/6J mice, aged 5-6 weeks, purchased from Guangdong Pharmachem Biotechnology Co.

(2) Test strain: A glycerol cryovial of the strain MNH18574 was thawed at 37 °C, and then inoculated onto an MM01 plate in an anaerobic workstation for activation. The activated strain was inoculated into MM01 liquid medium and anaerobically cultured to obtain a sufficient number of cultures. The cultured bacterial solution was centrifuged, concentrated, and then resuspended in a solvent to afford a test article with a purity and viable bacterial count ($2.5 \times 10^9$ - $4.0 \times 10^9$ CFU/mL) meeting the requirements of animal experiments.

(3) Negative control: PBS-Cys containing 25% glycerol.

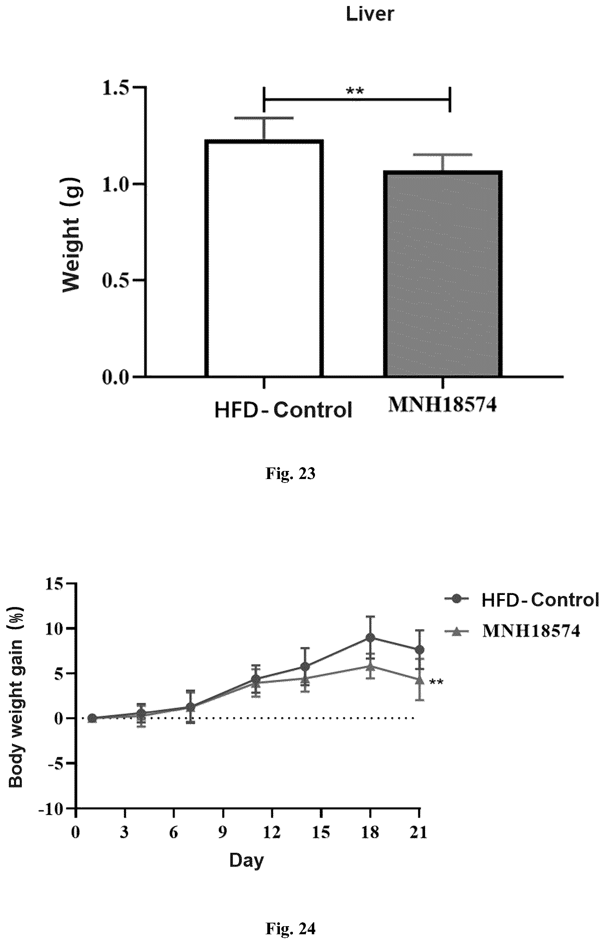

(4) Experimental procedure: Upon completion of the quarantine period, male C57BL/6J mice aged 5-6 weeks were fed with high-fat diet for 10 weeks. 16 mice with a body weight ranging from 35.50 g to 44.49 g were selected and randomized into 2 groups of 8 animals each (an experimental group and a control group) according to body weight. Gavage administration was initiated after the grouping (Day 1). The experimental group was administered with the MNH18574 bacterial solution in an amount of 0.1-0.3 mL/10 g of body weight, while the control group was given the negative control, twice a day, for 21 days. During the experiment, the mice were given *ad libitum* access to water and food under 12h/12h light/dark cycle. During the experiment, one general clinical observation was conducted after each virtual dosing or the end of the dosing. The animals were weighed on each Monday and Thursday during the dosing period, on the day of initial dosing (Day1), on Day 21 of dosing, and before dissection at the experimental endpoint. The endpoint of this experiment was the day following the end of dosing (Day 22). The animals were dissected and sampled according to the protocol at the experimental endpoint, and the data were summarized to analyze the body weight, the percent change in body weight, and the results of each dissection data. All data were expressed as mean $\pm$ SD, and plotted and statistically analyzed using the GraphPad Prism 8.0.2 software. For pairwise comparisons, t-test (Student's t test) was used. It was not indicated if there was no significance; and the significance of difference was indicated by *: *$p < 0.05$, **$p < 0.01$, ***$p < 0.001$, ****$p < 0.0001$. The results are shown in Fig. 23. MNH18574 can significantly reduce liver weight, indicating that MNH18574 can significantly ameliorate fatty liver caused by high-fat diet.

*4.2 In vivo* experiment of MNH18574 in a high-fat diet-induced obese mice model to verify the use of MNH18574 in the treatment and prevention of obesity and related diseases. This experiment has been ethically reviewed by the Laboratory Animal Care and Use Committee of Moon Biotech.

(1) Experimental animal: C57BL/6J mice, aged 5-6 weeks, purchased from Guangdong Pharmachem Biotechnology Co.

(2) Test strain: A glycerol cryovial of the strain MNH18574 was thawed at 37 °C, and then inoculated onto an MM01 plate in an anaerobic workstation for activation. The activated strain was inoculated into MM01 liquid medium and anaerobically cultured to obtain a sufficient number of cultures. The cultured bacterial solution was centrifuged, concentrated, and then resuspended in a solvent to afford a test article with a purity and viable bacterial count ($2.5 \times 10^9$ - $4.0 \times 10^9$ CFU/mL) meeting the requirements of animal experiments.

Negative control: PBS-Cys containing 25% glycerol.

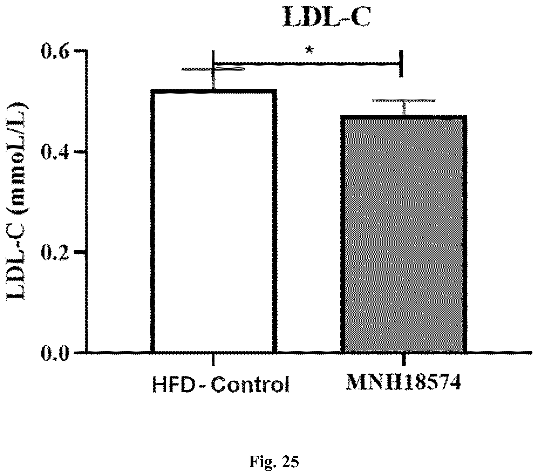

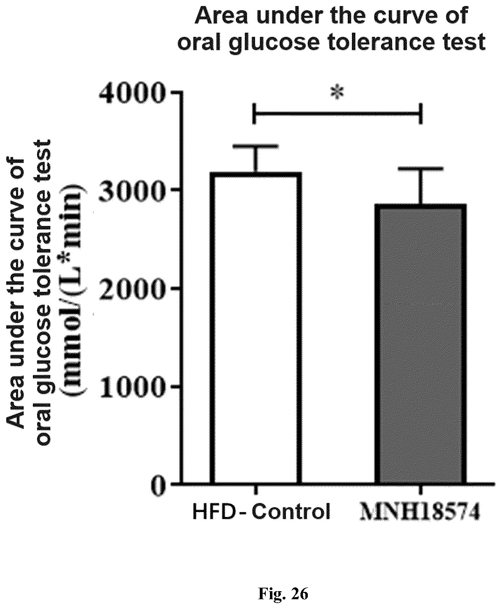

Experimental procedure: Upon completion of the quarantine period, male C57BL/6J mice aged 5-6 weeks were fed with high-fat diet for 10 weeks. 16 mice with a body weight ranging from 35.50 g to 44.49 g were selected and randomized into 2 groups of 8 animals each (an experimental group and a control group) according to body weight. Administration was initiated after the grouping (Day 1). The experimental group was administered with MNH18574, while the control group was given the negative control, twice a day, for 21 days. During the experiment, the mice were given *ad libitum* access to water and food under 12h/12h light/dark cycle. During the experiment, one general clinical observation was conducted after each virtual dosing or the end of the dosing. The animals were weighed on each Monday and Thursday during the dosing period, on the day of initial dosing (Day 1), on Day 21 of dosing, and before dissection at the experimental endpoint. The endpoint of this experiment was the day following the end of dosing (Day 22). The animals were dissected and sampled according to the protocol and their sera were tested at the experimental endpoint. The data were summarized to analyze the body weight, the percent change in body weight, and the results of each dissection data. All data were expressed as mean $\pm$ SD, and plotted and statistically analyzed using the GraphPad Prism 8.0.2 software. For pairwise comparisons, t-test (Student's t test) was used. It was not indicated if there was no significance; and the significance of difference was indicated by *: *$p < 0.05$, **$p < 0.01$, *** $p < 0.001$, **** $p < 0.0001$. The results are shown in Figs. 24-26. Fig. 24 shows that MNH18574 can significantly inhibit the body weight gain of high-fat diet-induced obese mice, suggesting an effect of preventing and treating obesity. The results in Fig. 25 show that MNH18574 can reduce the LDL cholesterol content in high-fat diet-induced obese mice, suggesting an effect of preventing and treating hyperlipidemia. The results in Fig. 26 show that MNH18574 can significantly reduce the area under the curve of oral glucose tolerance test (AUC of OGTT) in high-fat diet-induced obese mice, suggesting an effect of preventing and treating diabetes mellitus, and improving the blood glucose control function.

In summary, the strain intervention can effectively control total cholesterol and LDL cholesterol in blood. An elevated cholesterol level in plasma is closely related to the development of atherosclerosis. The strain of the present disclosure can reduce blood lipids, and has the effect of lowering relevant indicators of atherosclerosis-related diseases.

Finally, it should be noted that the above examples are only used to illustrate but not to limit the technical solutions of the present disclosure. Although the present disclosure has been described in detail with reference to the foregoing examples, those of ordinary skill in the art should understand that the technical solutions described in the foregoing examples can still be modified, or some or all of the technical features can be equivalently substituted. Such modifications or substitutions will not cause the essence of the corresponding technical solutions to deviate from the scope of technical solutions in the examples of the present disclosure.

## Claims

1. A microbial strain, wherein the microbial strain is a new species of the genus *Collinsella* (*Collinsella* sp.), and is useful for the prevention and/or treatment of a tumor; and wherein the nucleotide sequence of 16s rDNA of the microbial strain has a similarity of more than 98.65% to the nucleotide sequence as set forth in SEQ ID No: 1;
preferably, the nucleotide sequence of 16s rDNA of the microbial strain has a similarity of greater than or equal to 98.7%, 99%, 99.3%, 99.5%, 99.8%, 99.9%, or 100% to the nucleotide sequence as set forth in SEQ ID No: 1.

2. The microbial strain according to claim 1, wherein the microbial strain comprises *Collinsella* sp. MNH 18574, which was deposited with the Guangdong Microbial Culture Collection Center with a deposit name of *Collinsella* sp. MNH 18574 and a deposit number of GDMCC No: 62667;

    preferably, the microbial strain has an average nucleotide identity (ANI) value of at least 81.5% to the *Collinsella* strain with a deposit number of GDMCC No: 62667;
    preferably, the average nucleotide identity (ANI) value is 82%, 85%, 90%, 92%, 95%, 95.5%, 96%, 96.5%, 97%, 97.5%, 98%, 98.5%, 99%, 99.5%, 99.6%, 99.7%, 99.8%, 99.9%, or 100%.

3. A composition, comprising at least one of the microbial strain of claim 1 or 2, a fermentation broth, a supernatant of a fermentation broth, an extract from a fermentation broth, or a metabolite of said microbial strain;
preferably, the composition is provided in liquid or solid form.

4. The composition according to claim 4, wherein the pharmaceutical composition comprises $1 \times 10^4$ to $1 \times 10^{12}$ cfu/ml of said microbial strain.

5. The composition according to any one of claims 3 and 4, further comprising one or more pharmaceutically or food-acceptable carriers or excipients, and/or a probiotic.

6. The composition according to claim 5, wherein the excipient is at least one selected from a carrier, an excipient, a diluent, a lubricant, a wetting agent, an emulsifier, a suspension stabilizer, a preservative, a sweetener, and a flavor.

7. The composition according to claim 5, wherein the pharmaceutical composition is in one of the following forms: a powder, a microencapsulated powder, a capsule, a tablet, a lozenge, a granule, an oral liquid, a suspension, an emulsion, a liquid formulation, a sustained-release formulation, a nano-formulation, and a microencapsulated capsule.

8. The composition according to any one of claims 3 to 7, wherein the composition further comprises an immunosuppressive agent;

    optionally, the immunosuppressive agent comprises at least one of a PD-1 inhibitor, a CTLA-4 inhibitor, or a PD-L1 inhibitor;
    preferably, the immunosuppressive agent comprises at least one of an anti-PD-1 antibody, an anti-CTLA-4 antibody, or an anti-PD-L1 antibody;
    preferably, the anit-PD-L1 antibody is selected from durvalumab, atezolizumab, or avelumab;
    preferably, the anti-PD-1 antibody is selected from pembrolizumab or nivolumab;
    preferably, the anti-CTLA-4 antibody is selected from ipilimumab or tremelimumab.

9. The composition according to any one of claims 3 to 7, wherein the composition further comprises at least one of a glucose-lowering agent, a fat-reducing agent, a weight-reducing agent, or a GLP-1 receptor agonist; preferably, the glucose-lowering agent is metformin; and preferably, the fat-reducing agent or weight-reducing agent is at least one of liraglutide, dulaglutide, or semaglutide.

10. The composition according to any one of claims 3 to 8, wherein the composition is a drug product, a health care product, or a food product for preventing or treating a tumor, diabetes mellitus, obesity, a liver function disorder, a liver and kidney function disorder, inflammation, a metabolic disorder caused by obesity, a cardiovascular or cerebrovascular disease, hyperlipidemia, or atherosclerosis, and optionally comprises a pharmaceutically or food-acceptable carrier or excipient.

11. The composition according to any one of claims 3 to 8, and 10, wherein the tumor is selected from at least one of a solid tumor, a soft tissue tumor, a hematopoietic tumor, an adenoma, or a metastatic tumor;

    preferably, the tumor is a clinically refractory tumor or a drug-resistant tumor; more preferably, the clinically refractory tumor comprises a tumor with a low response to a PD-1 drug; the drug-resistant tumor comprises a tumor resistant to a PD-1 drug and/or a tumor resistant to a chemical agent, a chemotherapeutic agent, and/or an immunotherapeutic agent; and the clinically refractory tumor or drug-resistant tumor comprises at least one of lung cancer, liver cancer, and pancreatic cancer;

more preferably, the tumor comprises a tumor resistant to an immunotherapeutic agent, such as a tumor resistant to an anti-PD-1 antibody, an anti-CTLA-4 antibody, an anti-PD-L1 antibody, and/or a PD-L1 inhibitor;

preferably, the tumor is selected from one or more of: liver cancer, colon cancer, rectal cancer, colorectal cancer, lung cancer, breast cancer, cervical cancer, ovarian cancer, pancreatic cancer, bile duct cancer, kidney cancer, glioma, liposarcoma, chondrosarcoma, fibrosarcoma, melanoma, cervical cancer, neuroblastoma, lymphoma, leukemia, gastric cancer, hematological malignancy,

prostate cancer, small cell lung cancer, squamous cell carcinoma, head and neck tumor, bladder cancer, nasopharyngeal cancer, and multiple myeloma, and still preferably, the tumor is selected from one or more of: melanoma, glioma, head and neck tumor, lung cancer, colorectal cancer, bladder cancer, nasopharyngeal cancer, liver cancer, kidney cancer, pancreatic cancer, lymphoma (preferably cutaneous T-cell lymphoma or peripheral T-cell lymphoma), ovarian cancer, fibrosarcoma, and multiple myeloma.

12. The composition according to any one of claims 3 to 8, 10, and 11, wherein the composition prevents and/or treats a tumor by at least one of the following pathways: (a) inhibiting tumor volume growth; (b) inhibiting tumor weight increase; (c) inhibiting tumor cell growth; (d) improving the tumor response rate to a therapy; (e) improving the therapeutic efficacy of an immunosuppressive agent, e.g., improving the therapeutic efficacy of a PD-1 drug; (f) inhibiting tumor cell metastasis; (g) reducing resistance to a PD-1 drug; (h) inhibiting the tumor by at least one short-chain fatty acid or short-chain fatty acid salt of acetic acid or acetate, propionic acid or propionate, butyric acid or butyrate, valeric acid or valerate in SCFA; (i) inhibiting the tumor by regulating the subject's immune system to exert an immunomodulatory effect; or (j) promoting or activating the subject's immune system (e.g., increasing $CD4^+$ or $CD8^+$ T cell content) to kill the tumor or inhibit tumor growth.

13. Use of the strain of any one of claims 1 and 2 or the composition of any one of claims 3 to 12 in the manufacture of a medicament for preventing or treating a tumor, diabetes mellitus, obesity, a liver function disorder, a liver and kidney function disorder, inflammation, a metabolic disorder caused by obesity, a cardiovascular or cerebrovascular disease, hyperlipidemia, or atherosclerosis.

14. The use according to claim 13, wherein the diabetes mellitus is type II diabetes, insulin resistance syndrome, glucose intolerance, a complication of diabetic nephropathy, diabetic neuropathy, diabetic ophthalmopathy, or diabetic foot.

15. The use according to claim 13, wherein the liver function disorder is liver dysfunction caused by obesity, liver function impairment, NAFLD, or NASH.

16. The use according to claim 13, wherein the tumor is selected from at least one of a solid tumor, a soft tissue tumor, a hematopoietic tumor, an adenoma, or a metastatic tumor;

preferably, the tumor comprises a clinically refractory tumor or a drug-resistant tumor; more preferably, the clinically refractory tumor comprises a tumor with a low response to a PD-1 drug; the drug-resistant tumor comprises a tumor resistant to a PD-1 drug and/or a tumor resistant to a chemical agent, a chemotherapeutic agent, and/or an immunotherapeutic agent; and the clinically refractory tumor or drug-resistant tumor comprises at least one of lung cancer, liver cancer, and pancreatic cancer;

more preferably, the tumor comprises a tumor resistant to an immunotherapeutic agent, such as a tumor resistant to an anti-PD-1 antibody, an anti-CTLA-4 antibody, an anti-PD-L1 antibody, and/or a PD-L1 inhibitor;

preferably, the tumor is selected from one or more of: liver cancer, colon cancer, rectal cancer, colorectal cancer, lung cancer, breast cancer, cervical cancer, ovarian cancer, pancreatic cancer, bile duct cancer, kidney cancer, glioma, liposarcoma, chondrosarcoma, fibrosarcoma, melanoma, cervical cancer, neuroblastoma, lymphoma, leukemia, gastric cancer, hematological malignancy, prostate cancer, small cell lung cancer, squamous cell carcinoma, head and neck tumor, bladder cancer, nasopharyngeal cancer, and multiple myeloma, and still preferably, the tumor is selected from one or more of: melanoma, glioma, head and neck tumor, lung cancer, colorectal cancer, bladder cancer, nasopharyngeal cancer, liver cancer, kidney cancer, pancreatic cancer, lymphoma (preferably cutaneous T-cell lymphoma or peripheral T-cell lymphoma), ovarian cancer, fibrosarcoma, and multiple myeloma.

17. The use according to claim 13, wherein the tumor is prevented and/or treated by at least one of the following pathways: (a) inhibiting tumor volume growth; (b) inhibiting tumor weight increase; (c) inhibiting tumor cell growth; (d) improving the tumor response rate to a therapy; (e) improving the therapeutic efficacy of an immunosuppressive agent, e.g., improving the therapeutic efficacy of a PD-1 drug; (f) inhibiting tumor cell metastasis; (g) reducing the resistance to a PD-1 drug; (h) inhibiting the tumor by at least one short-chain fatty acid or short-chain fatty acid salt of acetic acid or acetate, propionic acid or propionate, butyric acid or butyrate, valeric acid or valerate in SCFA; (i) inhibiting the tumor by regulating the subject's immune system to exert an immunomodulatory effect; or (j) promoting or activating the subject's immune system (e.g., increasing CD4$^+$ or CD8$^+$ T cell content) to kill the tumor or inhibit tumor growth.

18. A method of treating a tumor, comprising administering to a subject the *Collinsella* strain of any one of claims 1 and 2 or a composition of any one of claims 3 to 8, 10, and 11.

19. A method of treating diabetes mellitus, obesity, a liver function disorder, a liver and kidney function disorder, inflammation, a metabolic disorder caused by obesity, a cardiovascular or cerebrovascular disease, hyperlipidemia, or atherosclerosis, comprising administering to a subject the *Collinsella* stain of any one of claims 1 and 2 or a composition of any one of claims 3-7, 9, and 10.

Fig. 1

Fig. 2

EP 4 640 823 A1

Fig. 3

## MNH18574-pH Tolerance

Fig. 4

Fig. 5

Fig. 6

**Fig. 7**

**Fig. 8**

Fig. 9

Fig. 10

**Fig. 11**

**Fig. 12**

## Tumor weight at endpoint

**Fig. 13**

## Tumor growth inhibition rate at endpoint

**Fig. 14**

## A (Negative control)

LLC1 (Lung cancer)
Non-response rate (TGI<40%): 75.00% (6/8)
Response rate (TGI>40%): 25.00% (2/8)

Average tumor volume in the Control group

40% TGI (vs. the Control group)

## B (Positive control)

LLC1 (Lung cancer)
Non-response rate (TGI<40%): 75.00% (6/8)
Response rate (TGI>40%): 25.00% (2/8)

Average tumor volume in the Control group

40% TGI (vs. the Control group)

## E (MNH18574&anti-PD-1)

LLC1 (Lung cancer)
Non-response rate (TGI<40%): 37.50% (3/8)
Response rate (TGI>40%): 62.50% (5/8)

Average tumor volume in the Control group

40% TGI (vs. the Control group)

Fig. 15

## Tumor volume

A (Negative control)
B (Positive control)
E (MNH18574&anti-PD-1)

**Fig. 16**

## Tumor volume at endpoint

A (Negative control) B (Positive control) E (MNH18574&anti-PD-1)

**Fig. 17**

Fig. 18

Fig. 19

**A (Negative control)**

KPC (Pancreatic cancer)
Non-response rate (TGI<40%): 100% (8/8)
Response rate (TGI>40%): 0.00% (0/8)

Average tumor volume in the Control group

40% TGI (vs. the Control group)

**B (Positive control)**

KPC (Pancreatic cancer)
Non-response rate (TGI<40%): 87.5% (7/8)
Response rate (TGI>40%): 12.50% (1/8)

Average tumor volume in the Control group

40% TGI (vs. the Control group)

**E (MNH18574&anti-PD-1)**

KPC (Pancreatic cancer)
Non-response rate (TGI<40%): 62.50% (5/8)
Response rate (TGI>40%): 37.50% (3/8)

Average tumor volume in the Control group

40% TGI (vs. the Control group)

**Fig. 20**

Fig. 21

Fig. 22

## Liver

**Fig. 23**

**Fig. 24**

Fig. 25

Fig. 26

## INTERNATIONAL SEARCH REPORT

| International application No. |
|---|
| **PCT/CN2024/070226** |

### A.    CLASSIFICATION OF SUBJECT MATTER

C12N1/20(2006.01)i;  A61P35/00(2006.01)i;  A61P3/06(2006.01)i;  C12R1/01(2006.01)n

According to International Patent Classification (IPC) or to both national classification and IPC

### B.    FIELDS SEARCHED

Minimum documentation searched (classification system followed by classification symbols)

IPC:C12N,A61P,C12R

Documentation searched other than minimum documentation to the extent that such documents are included in the fields searched

Electronic data base consulted during the international search (name of data base and, where practicable, search terms used)

Databases: VCN, VEN, WPABSC, ENTXT, OETXT, CNKI, PubMed, Elsevier Science , ISI WEB of Science; GenBank+E MBL; 中国专利生物序列检索系统, China Patent Biological Sequence Search System search terms: 对SEQ ID NO: 1的检索, search for SEQ ID NO: 1, 柯林斯菌, Collinsella, MNH 18574, GDMCC s 62667, 肿瘤, 癌, cancer, 血脂, cholestero, 血糖, blood glucose

### C.    DOCUMENTS CONSIDERED TO BE RELEVANT

| Category* | Citation of document, with indication, where appropriate, of the relevant passages | Relevant to claim No. |
|---|---|---|
| A | WO 2021138562 A1 (ASSEMBLY BIOSCIENCES INC.) 08 July 2021 (2021-07-08)<br>see embodiment 5, and abstract | 1-17 |
| A | CN 110049772 A (SHENZHEN BGI LIFE SCIENCES RESEARCH INSTITUTE) 23 July 2019 (2019-07-23)<br>see embodiments 4-5, and abstract | 1-17 |
| A | CN 111819295 A (MD HEALTHCARE INC.) 23 October 2020 (2020-10-23)<br>see abstract | 1-17 |
| A | 杨浩等 (YANG, Hao et al.). "Collinsella aerofaciens对高脂饮食小鼠糖脂代谢及肠道菌群的影响 (The Effect of Collinsella aerofaciens on Gluco-Lipid Metabolism and Gut Microbiota in High-fat Diet Mice)"<br>中国微生态学杂志 (Chinese Journal of Microecology),<br>Vol. 34, No. (11), 30 November 2022 (2022-11-30),<br>see abstract | 1-17 |

☑ Further documents are listed in the continuation of Box C.          ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **22 April 2024** | **30 April 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
|---|
| **PCT/CN2024/070226** |

| C. | DOCUMENTS CONSIDERED TO BE RELEVANT | |
|---|---|---|
| Category* | Citation of document, with indication, where appropriate, of the relevant passages | Relevant to claim No. |
| A | 杨浩等 (YANG, Hao et al.). "不同剂量Collinsella aerofaciens菌对C57BL/6J小鼠血糖血脂代谢水平的影响 (Effect of Collinsella aerofaciens on the Level of Blood Glucose and Lipid Metabolism in Mice)" <br> 医学研究杂志 (Journal of Medical Research), Vol. 51, No. (4), 30 April 2022 (2022-04-30), see abstract | 1-17 |

Form PCT/ISA/210 (second sheet) (July 2022)

**INTERNATIONAL SEARCH REPORT**

International application No.

**PCT/CN2024/070226**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

   a.  ☑  forming part of the international application as filed.

   b.  ☐  furnished subsequent to the international filing date for the purposes of international search (Rule 13*ter*.1(a)),

        ☐  accompanied by a statement to the effect that the sequence listing does not go beyond the disclosure in the international application as filed.

2.  ☐  With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT

| International application No. |
|---|
| **PCT/CN2024/070226** |

**Box No. II      Observations where certain claims were found unsearchable (Continuation of item 2 of first sheet)**

This international search report has not been established in respect of certain claims under Article 17(2)(a) for the following reasons:

1. ☑    Claims Nos.: **18-19**
because they relate to subject matter not required to be searched by this Authority, namely:

Claims 18-19 relate to the subject matter for treatment, which falls within the category of methods for treatment of the human or animal body, and falls within the cases set out in PCT Rule 39.1(iv) for which no international search is required. Therefore, no international search is performed.

2. ☐    Claims Nos.:
because they relate to parts of the international application that do not comply with the prescribed requirements to such an extent that no meaningful international search can be carried out, specifically:

3. ☐    Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
Information on patent family members

International application No.

**PCT/CN2024/070226**

| Patent document cited in search report | | | Publication date (day/month/year) | Patent family member(s) | | | Publication date (day/month/year) |
|---|---|---|---|---|---|---|---|
| WO | 2021138562 | A1 | 08 July 2021 | None | | | |
| CN | 110049772 | A | 23 July 2019 | WO | 2018107364 | A1 | 21 June 2018 |
| CN | 111819295 | A | 23 October 2020 | WO | 2019172604 | A1 | 12 September 2019 |
| | | | | US | 2023287517 | A1 | 14 September 2023 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **WUHAN PRICELLA**. Tumor cells: LLC1 mouse lung cancer cells. *Biotechnology Co., Ltd* (CL-0140)